(19) **Europäisches Patentamt / European Patent Office / Office européen des brevets**

(11) **EP 2 332 460 B1**

(12) # EUROPEAN PATENT SPECIFICATION

(45) Date of publication and mention
of the grant of the patent:
**05.06.2013 Bulletin 2013/23**

(51) Int Cl.:
**A61B 1/045** (2006.01)

(21) Application number: **11000358.9**

(22) Date of filing: **07.03.2006**

(54) **Biological observation apparatus**

Biologisches Beobachtungsgerät

Appareil d'observation biologique

(84) Designated Contracting States:
**DE FR GB**

(30) Priority: **13.05.2005 JP 2005141534**
**26.05.2005 JP 2005154372**

(43) Date of publication of application:
**15.06.2011 Bulletin 2011/24**

(62) Document number(s) of the earlier application(s) in
accordance with Art. 76 EPC:
**06715358.5 / 1 880 659**

(73) Proprietor: **Olympus Medical Systems Corp.**
**Tokyo 151-0072 (JP)**

(72) Inventors:
• **Gono, Kazuhiro**
**Tokyo 151-0072 (JP)**

• **Amano, Shoichi**
**Tokyo 151-0072 (JP)**
• **Takahashi, Tomoya**
**Tokyo 151-0072 (JP)**
• **Ohshima, Mutsumi**
**Tokyo 151-0072 (JP)**

(74) Representative: **Schicker, Silvia**
**Wuesthoff & Wuesthoff**
**Patent- und Rechtsanwälte**
**Schweigerstrasse 2**
**81541 München (DE)**

(56) References cited:
**EP-A1- 1 488 731     US-A- 5 408 263**
**US-A- 5 868 666     US-A1- 2003 158 470**

Printed by Jouve, 75001 PARIS (FR)

**Description**

Technical Field

The present invention relates to a biological observation apparatus that creates a quasi-narrowband filter through signal processing using a color image signal obtained by picking up an image of a living body, and displays the spectral image signal as a spectral image on a monitor.

Background Art

Conventionally, an endoscope apparatus that irradiates illumination light to obtain an endoscopic image inside a body cavity is widely used as a biological observation apparatus. An endoscope apparatus of this type uses an electronic endoscope having image pickup means that guides illumination light from a light source into a body cavity using a light guide or the like and which picks up a subject image from returning light thereof, and is arranged so that signal processing of an image pickup signal from the image pickup means is performed by a video processor in order to display an endoscopic image on an observation monitor for observing an observed region such as a diseased part.

One method of performing normal biological tissue observation using an endoscope apparatus involves emitting white light in the visible light range from a light source, irradiating frame sequential light on a subject via a rotary filter such as an RGB rotary filter, and obtaining a color image by performing synchronization and image processing on returning light of the frame sequential light by a video processor. In addition, another method of performing normal biological tissue observation using an endoscope apparatus involves positioning a color chip on a front face of an image pickup plane of image pickup means of an endoscope, emitting white light in the visible light range from a light source, picking up images by separating returning light of the frame sequential light at the color chip into each color component, and obtaining a color image by performing image processing by a video processor.

With biological tissue, absorption characteristics and scattering characteristics of light differ according to the wavelength of irradiated light. For example, Japanese Patent Laid-Open 2002-95635 proposes a narrowband light endoscope apparatus that irradiates illumination light in the visible light range on biological tissue as narrowband RGB frame sequential light having discrete spectral characteristics to obtain tissue information on a desired deep portion of the biological tissue.

In addition, Japanese Patent Laid-Open 2003-93336 proposes a narrowband light endoscope apparatus that performs signal processing on an image signal obtained from illumination light in the visible light range to create a discrete spectral image and to obtain tissue information on a desired deep portion of the biological tissue.

However, for example, with the apparatus described in above-mentioned Japanese Patent Laid-Open 2003-93336, while obtaining a spectral image through signal processing eliminates the need for a filter for creating narrowband RGB light, an obtained spectral image is merely outputted to a monitor. Therefore, there is a risk that an image displayed on the monitor is not an image whose color tone is suitable for observation of tissue information of a desired depth of biological tissue and that visibility is not favorable.

Furthermore, additional problems exist in the apparatus described in above-mentioned Japanese Patent Laid-Open 2003-93336 in that a configuration in which circuit systems are separated between normal images and spectral images result in a large circuit size, and although color adjustment and contour correction are performed on normal images, image processing such as color adjustment and contour correction are not performed on spectral images.

US 2003/0158470 A1 discloses a device for real-time screening of suspicious areas in images of a living body.

US 5,408,263 discloses an electronic endoscope apparatus having an arrangement whose discrimination circuit discriminates the type or the operational state of the light source in accordance with a discrimination signal transmitted from a discrimination signal generator, and, the processing operation to be performed by its video signal processing circuit is changed over to correspond to the difference in the type or the operational state of the light source. The video signal processing circuit performs a proper signal processing operation to correspond to the difference in the type of the light source to prevent deterioration in the image quality. The electronic endoscope apparatus is structured so that a different type light source can be adaptably connected to it.

US 5,868,666 discloses an endoscope apparatus including an endoscope a supply portion for supplying a required signal or energy to a peripheral unit relating to the endoscope, and a control portion for controlling the supply portion. The control portion is constituted to have at least one field programmable gate array and a function required by at least either of the endoscope or the peripheral unit relating to the endoscope is realized by receiving circuit information for constituting a control circuit for controlling the supply portion to selectively write the circuit information on the field programmable gate array so that the control circuit is constituted.

Accordingly, the present invention has been made in consideration of the above circumstances, and an object thereof is to provide a biological observation apparatus capable of adjusting tissue information of a desired depth of biological tissue based on a spectral image obtained through signal processing to image information having a color tone suitable for observation, and at the same time, improving image quality of a signal to be displayed/outputted in order to attain favorable visibility.

Another object of the present invention is to provide a biological observation apparatus capable of adjusting tissue information of a desired depth of biological tissue based on a spectral image obtained through signal processing to image information having a color tone suitable for observation, and at the same time, capable of suppressing circuit size and sharing circuits for performing necessary signal processing such as white balance and γ adjustment.

Disclosure of Invention

Means for Solving the Problem

The above defined problem is solved by a biological observation apparatus as defined in claim 1.

A biological observation apparatus comprises: an illuminating section that irradiates light to a living body that is a subject to be examined; an image pickup section that photoelectrically converts light reflected from the living body based on the irradiating light and creates an image pickup signal; and a signal processing control section that controls operations of the illuminating section and/or the image pickup section and outputs the image pickup signal to a display device, wherein the signal processing control section includes: a spectral signal creating section that creates a spectral signal corresponding to an optical wavelength narrowband image from the image pickup signal through signal processing; a color adjusting section that, when outputting the spectral signal to the display device, allocates a different color tone for each of a plurality of bands forming the spectral signal; and an image quality adjusting section that adjusts image quality of a signal to be outputted to the display device.

In addition, a biological observation apparatus according to another aspect of the present invention comprises: an illuminating section that irradiates light to a living body that is a subject to be examined; an image pickup section that photoelectrically converts light reflected from the living body based on the irradiating light and creates an image pickup signal; and a signal processing control section that controls operations of the illuminating section and/or the image pickup section and outputs the image pickup signal to a display device, wherein the signal processing control section includes: a spectral signal creating section that creates a spectral signal corresponding to an optical wavelength narrowband image from the image pickup signal through signal processing; and a color adjusting section that, when outputting the spectral signal to the display device, allocates a different color tone for each of a plurality of bands forming the spectral signal, further wherein, with the exception of at least the spectral signal creating section and the color adjusting section, the other signal processing sections are shared for respective signal processing of the image pickup signal and of the spectral signal.

Brief Description of the Drawings

Fig. 1 is a conceptual diagram showing a flow of signals when creating a spectral image signal from a color image signals according to a first example useful for understanding the present invention;
Fig. 2 is a conceptual diagram showing integrating computation of a spectral image signal according to the first example;
Fig. 3 is a conceptual diagram showing an external appearance of a biological observation apparatus according to the first example;
Fig. 4 is a block diagram showing a configuration of the biological observation apparatus shown in Fig. 3;
Fig. 5 is an exterior view of a chopper shown in Fig. 4;
Fig. 6 is a diagram showing an array of color filters positioned on an image pickup plane of a CCD shown in Fig. 4;
Fig. 7 is a diagram showing spectral sensitivity characteristics of the color filters shown in Fig. 6;
Fig. 8 is a configuration diagram showing a configuration of a matrix computing section shown in Fig. 4;
Fig. 9 is a spectrum diagram showing a spectrum of a light source according to the first example;
Fig. 10 is a spectrum diagram showing a reflectance spectrum of a living body according to the first example;
Fig. 11 is a diagram showing a layer-wise structure of biological tissue to be observed by the biological observation apparatus shown in Fig. 4;
Fig. 12 is a diagram describing layer-wise reached states in biological tissue of an illumination light from the biological observation apparatus shown in Fig. 4;
Fig. 13 is a diagram showing spectral characteristics of respective bands of white light;
Fig. 14 is a first diagram showing respective band images by the white light of Fig. 13;
Fig. 15 is a second diagram showing respective band images by the white light of Fig. 13;
Fig. 16 is a third diagram showing respective band images by the white light of Fig. 13;

Fig. 17 is a diagram showing spectral characteristics of a spectral image created at the matrix computing section shown in Fig. 8;

Fig. 18 is a first diagram showing respective spectral images of Fig. 17;

Fig. 19 is a second diagram showing respective spectral images of Fig. 17;

Fig. 20 is a third diagram showing respective spectral images of Fig. 17;

Fig. 21 is a block diagram showing a configuration of a color adjusting section shown in Fig. 4;

Fig. 22 is a diagram describing operations of the color adjusting section shown in Fig. 21;

Fig. 23 is a block diagram showing a configuration of a modification of the color adjusting section shown in Fig. 4;

Fig. 24 is a diagram showing spectral characteristics of a first modification of the spectral image shown in Fig. 17;

Fig. 25 is a diagram showing spectral characteristics of a second modification of the spectral image shown in Fig. 17;

Fig. 26 is a diagram showing spectral characteristics of a third modification of the spectral image shown in Fig. 17;

Fig. 27 is a block diagram showing another configuration example of the matrix computing section according to the first example;

Fig. 28 is a block diagram showing a configuration of a biological observation apparatus according to a second example useful for understanding the present invention;

Fig. 29 is a diagram showing an example of a light quantity control section in a biological observation apparatus according to a fourth example useful for understanding the present invention;

Fig. 30 is a diagram showing another example of the light quantity control section;

Fig. 31 is a diagram showing yet another example of the light quantity control section;

Fig. 32 is a block diagram showing a configuration of the biological observation apparatus according to the fourth example;

Fig. 33 is a diagram showing charge accumulation times of a CCD shown in Fig. 32;

Fig. 34 is a diagram that is a modification of Fig. 32 and which shows charge accumulation times of the CCD;

Fig. 35 is a diagram showing an example of image quality improvement in a biological observation apparatus according to an eighth example useful for understanding the present invention;

Fig. 36 is a diagram showing an example of image quality improvement in a biological observation to a observation apparatus according to a ninth example useful for understanding the present invention;

Fig. 37 is a diagram showing another example of image quality improvement in the biological observation apparatus according to the ninth example;

Fig. 38 is a diagram showing an example of image quality improvement in a biological observation apparatus according to a tenth example useful for understanding the present invention;

Fig. 39 is a diagram showing an example of image quality improvement in a biological observation apparatus according to a twelfth example useful for understanding the present invention;

Fig. 40 is a diagram showing another example of image quality improvement in the biological observation apparatus according to the twelfth example;

Fig. 41 is a diagram showing yet another example of image quality improvement in the biological observation apparatus according to the twelfth example;

Fig. 42 is a block diagram showing a configuration of a biological observation apparatus according to a thirteenth example useful for understanding the present invention;

Fig. 43 is a block diagram showing a configuration of a biological observation apparatus according to a fourteenth example useful for understanding the present invention;

Fig. 44 is a block diagram showing a configuration of a biological observation apparatus according to a fifteenth example useful for understanding the present invention;

Fig. 45 is a diagram showing an array of color filters in a biological observation apparatus according to a sixteenth example useful for understanding the present invention;

Fig. 46 is a diagram showing spectral sensitivity characteristics of the color filters shown in Fig. 45; and

Fig. 47 is a flowchart during matrix computation in a biological observation apparatus useful for understanding the present invention.

Best Mode for Carrying Out the Invention

Example useful for understanding the present invention will now be described with reference to the drawings.

[First example]

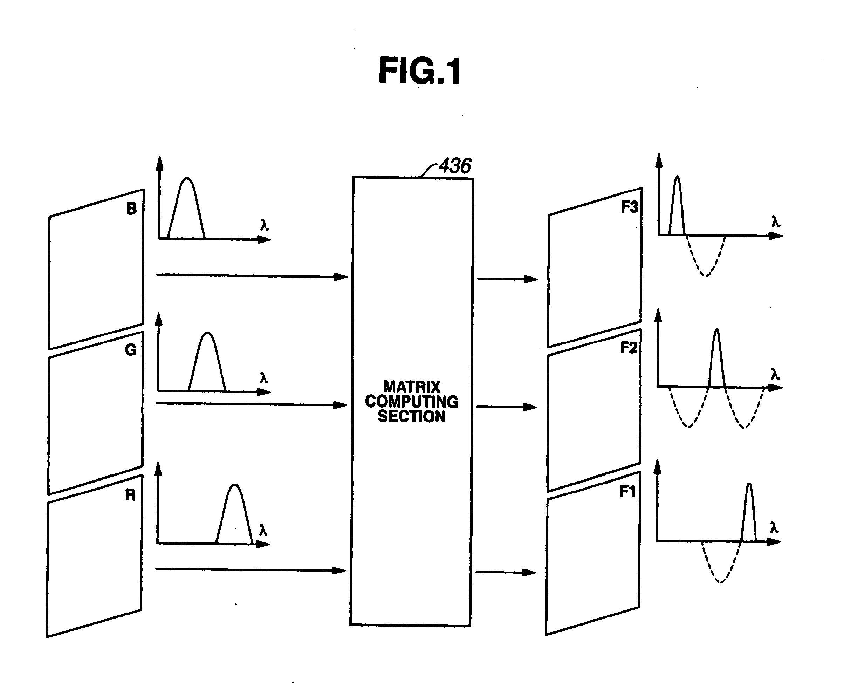

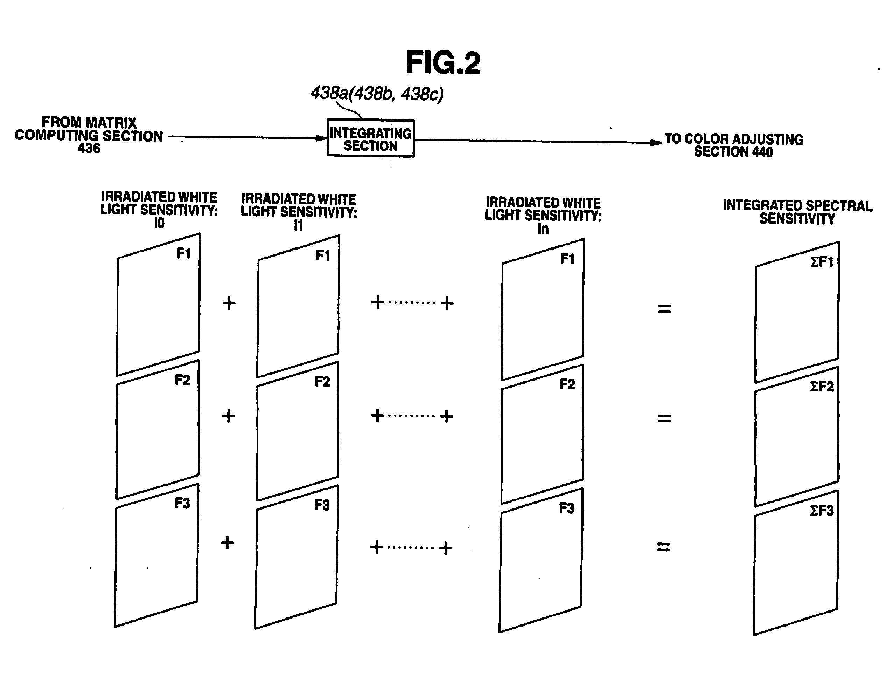

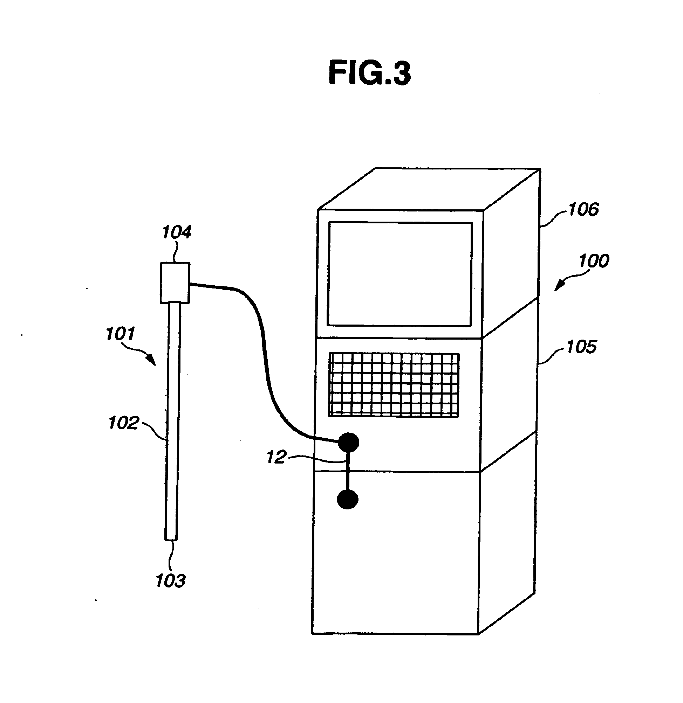

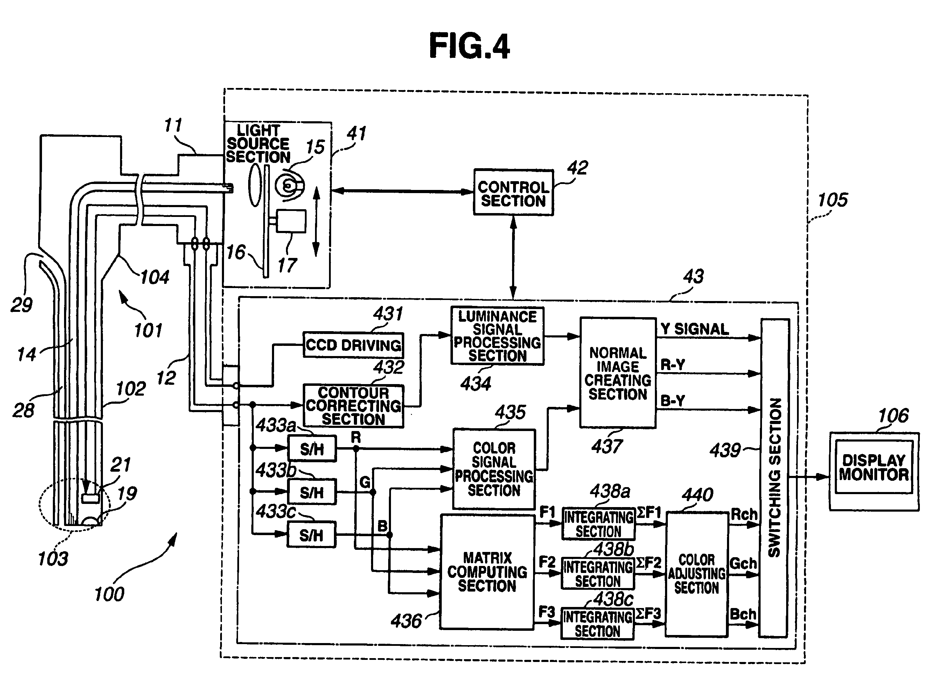

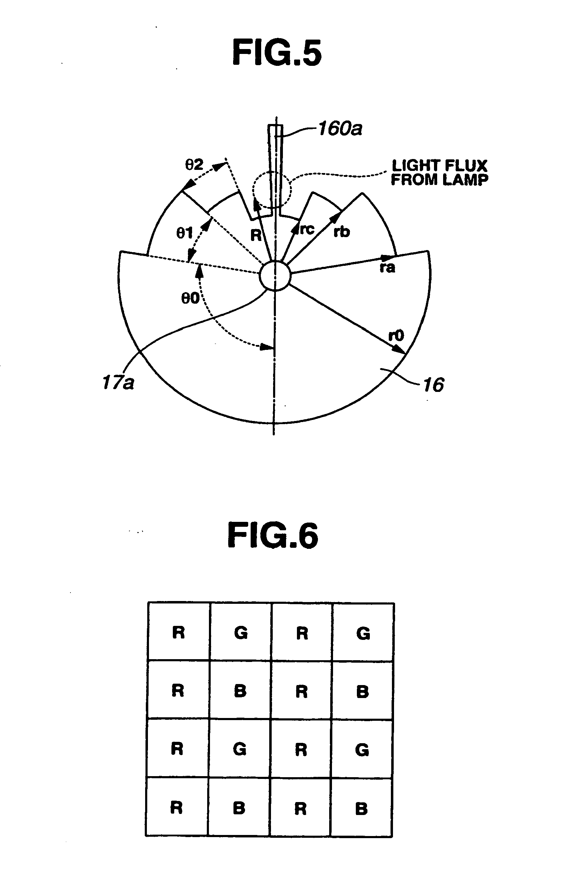

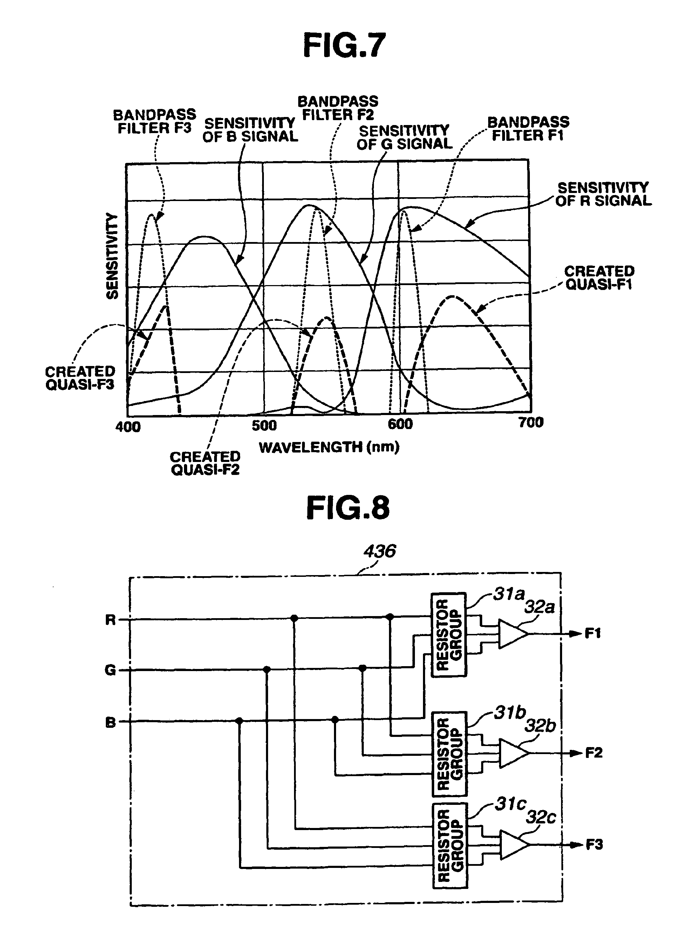

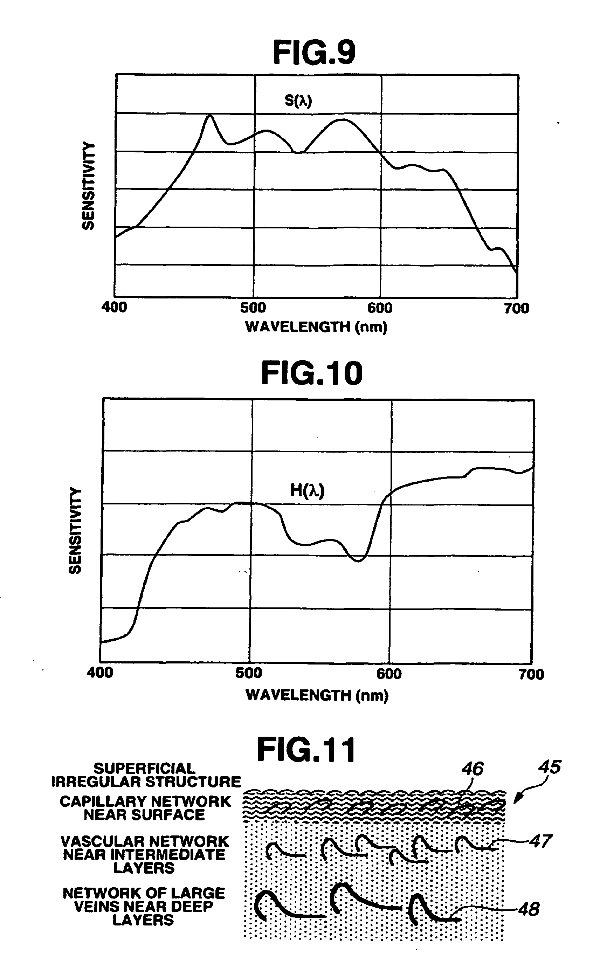

Figs. 1 to 26 relate to a first example useful for understanding the present invention, wherein: Fig. 1 is a conceptual diagram showing a flow of signals when creating a spectral image signal from a color image signal; Fig. 2 is a conceptual diagram showing integrating computation of a spectral image signal; Fig. 3 is an external view showing an external appearance of an electronic endoscope apparatus; Fig. 4 is a block diagram showing a configuration of the electronic endoscope apparatus shown in Fig. 3; Fig. 5 is an exterior view of a chopper shown in Fig. 4; Fig. 6 is a diagram showing an array of color filters positioned on an image pickup plane of a CCD shown in Fig. 3; Fig. 7 is a diagram showing spectral sensitivity characteristics of the color filters shown in Fig. 6; Fig. 8 is a configuration diagram showing a configuration of a matrix computing section shown in Fig. 4; Fig. 9 is a spectrum diagram showing a spectrum of a light source; and Fig. 10 is a spectrum diagram showing a reflectance spectrum of a living body.

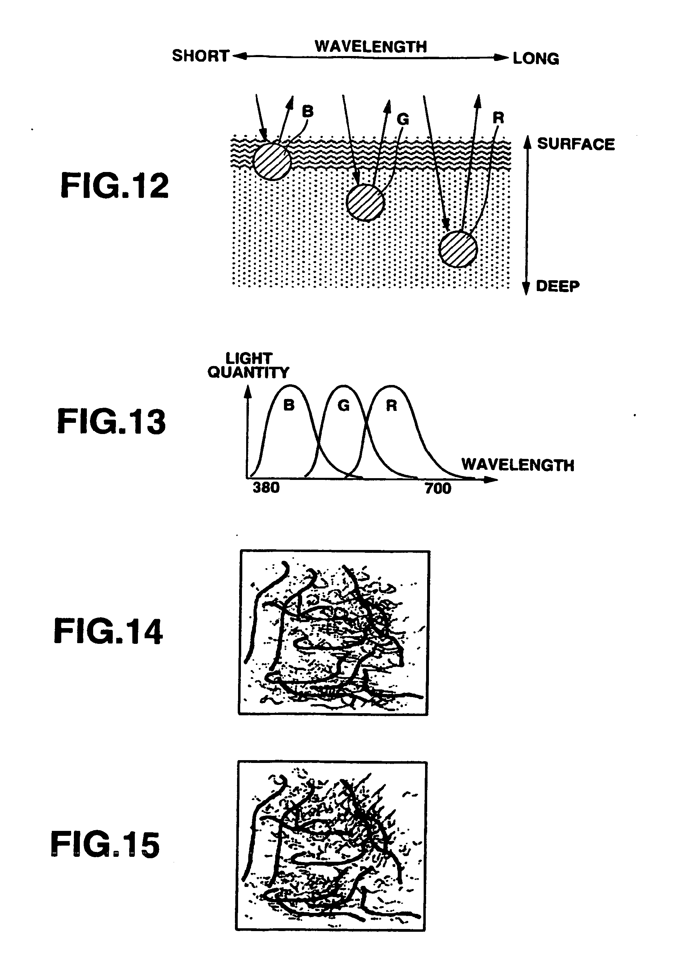

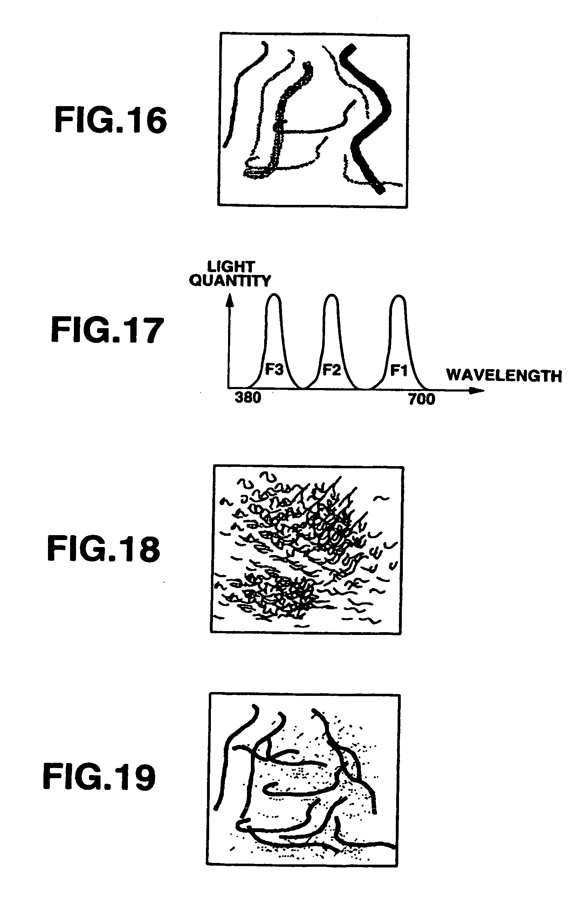

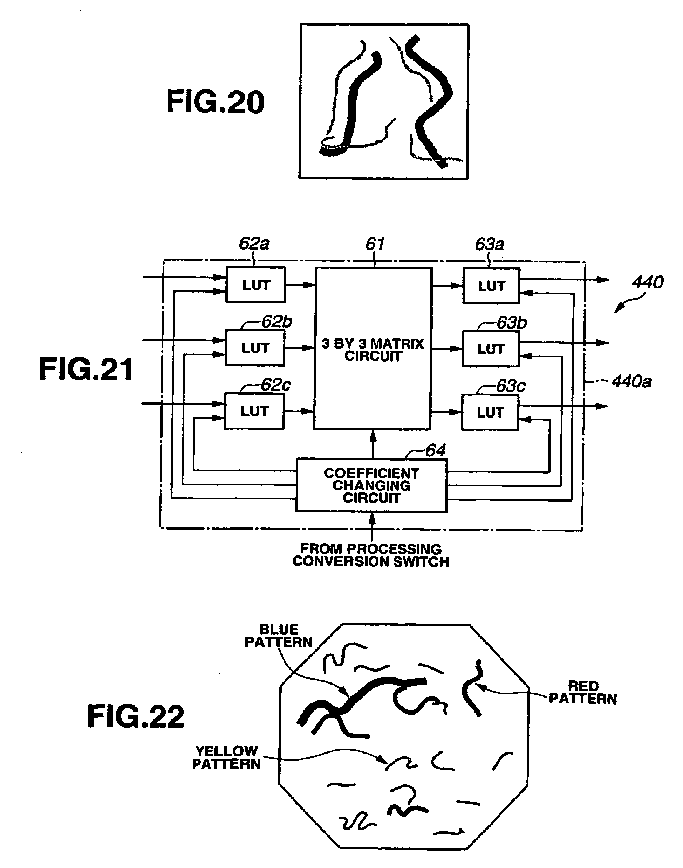

Fig. 11 is a diagram showing a layer-wise structure of biological tissue to be observed by the electronic endoscope apparatus shown in Fig. 4; Fig. 12 is a diagram describing reached states in a layer-wise direction in biological tissue of an illumination light from the electronic endoscope apparatus shown in Fig. 4; Fig. 13 is a diagram showing spectral characteristics of respective bands of white light; Fig. 14 is a first diagram showing respective band images by the white light shown in Fig. 13; Fig. 15 is a second diagram showing respective band images by the white light shown in Fig. 13; Fig. 16 is a third diagram showing respective band images by the white light shown in Fig. 13; Fig. 17 is a diagram showing spectral characteristics of a spectral image created by the matrix computing section shown in Fig. 8; Fig. 18 is a first diagram showing respective spectral images shown in Fig. 17; Fig. 19 is a second diagram showing respective spectral images shown in Fig. 17; and Fig. 20 is a third diagram showing respective spectral images shown in Fig. 17.

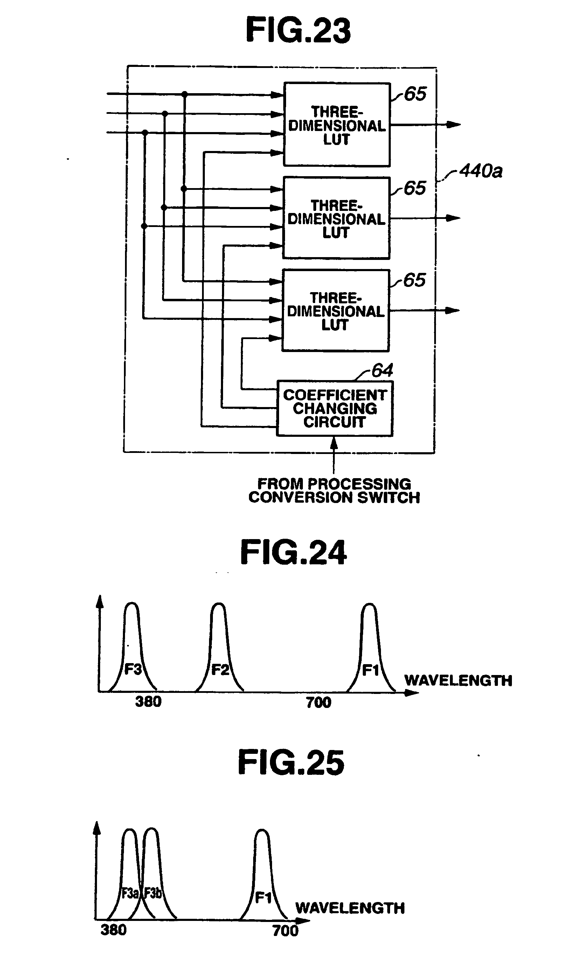

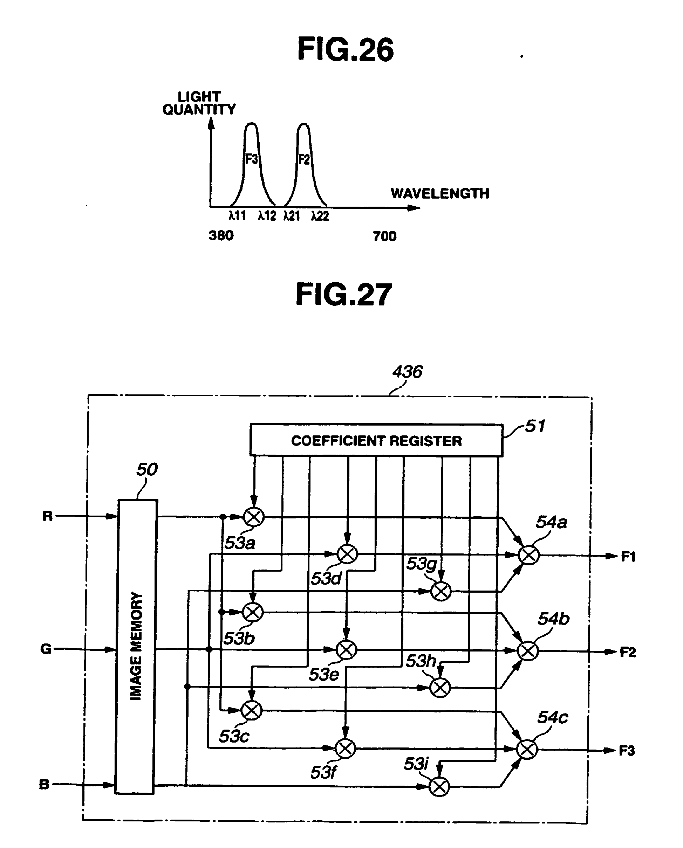

Fig. 21 is a block diagram showing a configuration of a color adjusting section shown in Fig. 4; Fig. 22 is a diagram describing operations of the color adjusting section shown in Fig. 21; Fig. 23 is a block diagram showing a configuration of a modification of the color adjusting section shown in Fig. 4; Fig. 24 is a diagram showing spectral characteristics of a first modification of the spectral image shown in Fig. 17; Fig. 25 is a diagram showing spectral characteristics of a second modification of the spectral image shown in Fig. 17; and Fig. 26 is a diagram showing spectral characteristics of a third modification of the spectral image shown in Fig. 17.

An electronic endoscope apparatus as a biological observation apparatus according to examples useful for understanding the present invention irradiates light from an illuminating light source to a living body that is a subject to be examined, receives light reflected from the living body based on the irradiating light at a solid state image pickup device that is an image pickup section and creates an image pickup signal that is a color image signal by photoelectrically converting the signal, and creates from the image pickup signal through signal processing a spectral image signal that is a spectral image corresponding to an optical wavelength narrowband image.

Before presenting a description on the first embodiment of the present invention, a matrix calculating method that forms the foundation of the present invention will be described below. In this case, "matrix" refers to a predetermined coefficient used when creating a spectral image signal from a color image signal obtained in order to create a color image (hereinafter referred to as a normal signal).

In addition, following the description on a matrix, a correcting method for obtaining a more accurate spectral image signal and an S/N ratio improving method that enhances the S/N ratio of a created spectral image signal will be described. The correcting method and the S/N ratio improving method are to be used as needed. Furthermore, in the following description, vectors and matrices shall be denoted using bold characters or o (for example, matrix A shall be denoted as "bold A" or "<A>"). Other mathematical concepts shall be denoted without character decoration.

(Matrix calculating method)

Fig. 1 is a conceptual diagram showing a flow of signals when creating a spectral image signal to an image having a narrowband optical wavelength from a color image signal (in this case, while R/G/B will be used for simplicity, a combination of G/Cy/Mg/Ye may also be used with a complementary type solid state image pickup device as is the case in an example to be described later).

First, the electronic endoscope apparatus converts the respective color sensitivity characteristics of R/G/B into numerical data. In this case, color sensitivity characteristics of R/GB refer to the output characteristics of wavelengths respectively obtained when using a white light source to pickup an image of a white subject.

The respective color sensitivity characteristics of R/G/B are displayed on the right hand side of each image data as a simplified graph. In addition, the respective R/GB color sensitivity characteristics at this point are assumed to be n-dimension column vectors <R>/<G>/<B>.

Next, the electronic endoscope apparatus converts into numerical data the characteristics of narrow bandpass filters F1/F2/F3 for spectral images to be extracted (as a priori information, the electronic endoscope apparatus is aware of characteristics of filters capable of efficiently extracting structures; as for the characteristics of the filters, it is assumed that the passbands of the respective filters are wavelength ranges of approximately 590 nm to 610 nm, approximately 530 nm to 550 nm and approximately 400 nm to 430 nm).

In this case, "approximately" is a concept that includes around $\pm 10$ nm as far as wavelengths are concerned. The respective filter characteristics at this point are assumed to be n-dimension column vectors <F1>/<F2>/<F3>. Based on the obtained numerical data, an optimum coefficient set approximating the following relationship is determined. In other words, determining elements of a matrix satisfying

$$(R \quad G \quad B)\begin{pmatrix} a_1 & a_2 & a_3 \\ b_1 & b_2 & b_3 \\ c_1 & c_2 & c_3 \end{pmatrix} = (F_1 \quad F_2 \quad F_3) \qquad \cdots(1)$$

shall suffice.

The solution of the optimization proposition presented above is obtained as follows. If <C> denotes a matrix representing color sensitivity characteristics of R/G/B, <F> denotes spectral characteristics of a narrow bandpass filter to be extracted, and <A> denotes a coefficient matrix to be determined, it follows that

$$C = (R \quad G \quad B) \qquad A = \begin{pmatrix} a_1 & a_2 & a_3 \\ b_1 & b_2 & b_3 \\ c_1 & c_2 & c_3 \end{pmatrix} \qquad F = (F_1 \quad F_2 \quad F_3) \qquad \cdots(2)$$

Therefore, the proposition expressed as Formula 1 is equivalent to determining a matrix <A> that satisfies the following relationship.

$$CA = F \qquad \cdots(3)$$

Here, since n>3 is true for n-number of dots in a sequence as spectral data representing spectral characteristics, Formula 3 is obtained as a solution of linear least squares method instead of a linear simultaneous equation. In other words, deriving a pseudo inverse matrix from Formula 3 shall suffice. Assuming that a transposed matrix of the matrix <C> is <$^t$C>, Formula 3 may be expressed as

$$^tCCA = {^t}CF \qquad \cdots(4)$$

Since <$^t$CC> is an n by n square matrix, Formula 4 may be viewed as a simultaneous equation on the matrix <A>, whereby a solution thereof may be determined from

$$A = ({^t}CC)^{-1}{^t}CF \qquad \cdots(5)$$

By transforming the left hand side of Formula 3 with respect to the matrix <A> determined by Formula 5, the electronic endoscope apparatus is able to approximate the characteristics of the narrow bandpass filters F1/F2/F3 to be extracted. This concludes the description on the matrix calculating method that forms the foundation of the present invention.

Using a matrix calculated in this manner, a matrix computing section 436, to be described later, normally creates a spectral image signal from a color image signal.

(Correcting method)

Next, a correcting method for obtaining a more accurate spectral image signal will be described.

In the description of the matrix calculating method presented above, the method is accurately applied in a case where a light flux received by a solid state image pickup device such as a CCD is perfect white light (all wavelength intensities are the same in the visible range). In other words, optimum approximation is achieved when the respective outputs of R, G and B are the same.

However, in real-world endoscopic observation, since an illuminated light flux (light flux from a light source) is not perfect white light nor is the reflectance spectrum of a living body uniform, the light flux received by a solid state image pickup device is also not white light (coloration suggests that the R, G and B values are not the same).

Therefore, in actual processing, in order to more accurately solve the proposition expressed by Formula 3, it is desirable to take spectral characteristics of illumination light and reflection characteristics of a living body into consideration in addition to RGB color sensitivity characteristics.

Let us now assume that the color sensitivity characteristics are respectively $R(\lambda)$, $G(\lambda)$ and $B(\lambda)$, an example of the spectral characteristics of illumination light is $S(\lambda)$, and an example of the reflection characteristics of a living body is $H(\lambda)$. Incidentally, the spectral characteristics of illumination light and the reflection characteristics of a living body need not necessarily be the characteristics of the apparatus to be used for examination or the subject to be examined, and, for example, general characteristics obtained in advance may be used instead.

Using these coefficients, correction coefficients kR/kG/kB may be determined by

$$k_R = \left( \int S(\lambda) \times H(\lambda) \times R(\lambda) d\lambda \right)^{-1}$$
$$k_G = \left( \int S(\lambda) \times H(\lambda) \times G(\lambda) d\lambda \right)^{-1}$$
$$k_B = \left( \int S(\lambda) \times H(\lambda) \times B(\lambda) d\lambda \right)^{-1} \qquad \cdots (6)$$

A sensitivity correction matrix denoted by <K> may be determined as follows.

$$\mathbf{K} = \begin{pmatrix} k_R & 0 & 0 \\ 0 & k_G & 0 \\ 0 & 0 & k_B \end{pmatrix} \qquad \cdots (7)$$

Therefore, as for the coefficient matrix <A>, the addition of the correction represented by Formula 7 to Formula 5 results in the following.

$$\mathbf{A'} = \mathbf{KA} = \mathbf{K}({}'\mathbf{CC})^{-1}{}'\mathbf{CF} \qquad \cdots (8)$$

In addition, when performing actual optimization, taking advantage of the fact that 0 replaces negative spectral sensitivity characteristics of targeted filters (F1/F2/F3 in Fig. 1) during image display (in other words, only portions having positive sensitivity among the spectral sensitivity characteristics of filters are used), an allowance for portions of an optimized sensitivity distribution becoming negative is added. In order to create narrowband spectral sensitivity characteristics from broad spectral sensitivity characteristics, the electronic endoscope apparatus can create a component that approximates a band having sensitivity by adding negative sensitivity characteristics to the targeted characteristics of F1/F2/F3 as shown in Fig. 1.

(S/N ratio improving method)

Next, a description will be given on a method for enhancing the S/N ratio and accuracy of a created spectral image signal. Through the addition of the above-described processing method, the S/N ratio improving method further solves the following problems.

(i) When any of original signals (R/G/B) in the above-described matrix calculating method temporarily enters a saturated state, there is a possibility that the characteristics of the filters F1 to F3 in the processing method differ significantly from characteristics (ideal characteristics) of a filter capable of efficiently extracting a structure (when created only from two signals among R/G/B, it is required that neither of the two original signals are saturated).

(ii) Since a narrowband filter is created from a broadband filter when converting a color image signal into a spectral image signal, sensitivity degradation occurs, resulting in the creation of a smaller spectral image signal component and inferior S/N ratio.

With the present S/N ratio improving method, as shown in Fig. 2, illumination light is irradiated in several stages (e.g., n-stages, where n is an integer equal to or greater than 2) through 1 field (1frame) of a normal image (an ordinary color image) (irradiation intensity may be varied for each stage; in Fig. 2, the stages are denoted by reference characters 10 to In; this procedure can be achieved wholly by controlling illumination light).

Consequently, the electronic endoscope apparatus can reduce illumination intensity for each stage, thereby suppressing occurrences of saturated states in the respective R, G and B signals. In addition, image signals separated into several stages are added n-times at a post-stage. As a result, the electronic endoscope apparatus is able to increase the signal component to enhance S/N ratio. In Fig. 2, integrating sections 438a to 438c function as image quality adjusting sections that improve S/N ratio.

This concludes the descriptions on the matrix calculating method that forms the foundation of the present invention, as well as the correcting method for determining an accurate and executable spectral image signal and the method for enhancing the S/N ratio of a created spectral image signal.

A modification of the above-described matrix calculating method will now be described.

(Modification of matrix calculating method)

Let us assume that color image signals are denoted as R, G, B, and spectral image signals to be estimated as F1, F2 and F3. More precisely, although color image signals R, G, B are functions of a position x,y on an image and therefore, for example, it should be denoted as R(x,y), such notations shall be omitted herein.

An objective is to estimate a 3 by 3 matrix <A> that calculates F1, F2 and F3 from R, G, and B. Once <A> is estimated, it is now possible to calculate F1, F2 and F3 from R, G, B using Formula 9 below.

$$\begin{pmatrix} F_1 \\ F_2 \\ F_3 \end{pmatrix} = A \begin{pmatrix} R \\ G \\ B \end{pmatrix} \quad \cdots (9)$$

Notation of the following data will now be defined. Spectral characteristics of a subject to be examined: H($\lambda$), <H> = (H($\lambda$1), H($\lambda$2), ...H($\lambda$n))$^t$, where $\lambda$ denotes wavelength and t denotes transposition in matrix computation. In a similar manner, spectral characteristics of illumination light: S($\lambda$), <S> = (S($\lambda$1), S($\lambda$2), ...S($\lambda$n))$^t$, spectral sensitivity characteristics of a CCD: J($\lambda$), <J> = (J($\lambda$1), J($\lambda$2), ...J($\lambda$n))t, spectral characteristics of filters performing color separation: in the case of primary colors

$$R(\lambda), <R> = (R(\lambda 1), R(\lambda 2), \ldots R(\lambda n))^t,$$

$$G(\lambda), <G> = (G(\lambda 1), G(\lambda 2), \ldots G(\lambda n))^t,$$

and

$$B(\lambda), <B> = (B(\lambda 1), B(\lambda 2), \ldots B(\lambda n))^t.$$

As indicated by Formula 10, <R>, <G> and <B> can be bundled together into a matrix <C>.

$$C = \begin{pmatrix} R \\ G \\ B \end{pmatrix} \quad \cdots(10)$$

Image signals R, G, B and spectral signals F1, F2 and F3 may be expressed by matrix as follows.

$$P = \begin{pmatrix} R \\ G \\ B \end{pmatrix}, \qquad Q = \begin{pmatrix} F_1 \\ F_2 \\ F_3 \end{pmatrix} \quad \cdots(11)$$

An image signal <P> may be calculated using the following formula.

$$P = CSJH \quad \cdots(12)$$

Assuming now that a color separation filter for obtaining <Q> is denoted as <F>, in the same manner as Formula 12,

$$Q = FSJH \quad \cdots(13)$$

At this point, as a first important hypothesis, if it is assumed that the spectral reflectance of the subject to be examined may be expressed as a linear sum of three elementary spectral characteristics, <H> may be expressed as

$$H = DW \quad \cdots(14)$$

where <D> denotes a matrix having three elementary spectrums D1($\lambda$), D2($\lambda$), D3($\lambda$) as column vectors and <W> denotes a weighting coefficient representing the contribution of D1($\lambda$), D2($\lambda$), D3($\lambda$) towards <H>. It is known that the above approximation is true when the color tone of the subject to be examined does not vary significantly.

Assigning Formula 14 into Formula 12 we obtain

$$P = CSJH = CSJDW = MW \quad \cdots(15)$$

where the 3 by 3 matrix <M> represents a matrix in which the calculation results of matrices <CSJD> are bundled together.

In the same manner, assigning Formula 14 into Formula 13 we obtain

$$Q = FSJH = FSJDW = M'W \quad \cdots(16)$$

where, similarly, the 3 by 3 matrix <M'> represents a matrix in which the calculation results of matrices <FSJD> are bundled together.

Ultimately, eliminating <W> from Formulas 15 and 16 we obtain

$$Q = M'M^{-1}P \qquad \cdots(17)$$,

where <M⁻¹> represents an inverse matrix of matrix <M>. Ultimately, <M', M⁻¹> turns out to be a 3 by 3 matrix which becomes the estimation target matrix <A>.

At this point, as a second important hypothesis, when performing color separation using a bandpass filter, let us assume that the spectral characteristics of the subject to be examined within the band may be approximated using a single numerical value. In other words,

$$H = (h_1, h_2, h_3)^t \qquad \cdots(18)$$.

If the hypothesis is true when also taking into consideration a case where the bandpass for color separation is not a perfect bandpass and may have sensitivity in other bands, a matrix similar to that of Formula 17 can be ultimately estimated by considering the <W> in Formulas 15 and 16 as the above-described <H>.

Next, a specific configuration of an electronic endoscope apparatus in the first example useful for understanding the present invention will be described with reference to Fig. 3. Incidentally, the other examples described below may be similarly configured.

As shown in Fig. 3, an electronic endoscope apparatus 100 comprises an endoscope 101, an endoscope apparatus main body 105, and a display monitor 106 as a display device. In addition, the endoscope 101 is primarily constituted by: an insertion portion 102 to be inserted into the body of a subject to be examined; a distal end portion 103 provided at a distal end of the insertion portion 102; and an angle operating section 104 provided on an opposite side of the distal end side of the insertion portion 102 and which is provided for performing or instructing operations such as bending operations of the distal end portion 103.

An image of the subject to be examined acquired by the endoscope 101 is subjected to predetermined signal processing at the endoscope apparatus main body 105, and a processed image is displayed on the display monitor 106.

Next, the endoscope apparatus main body 105 will be described in detail with reference to Fig. 4. Fig. 4 is a block diagram of the simultaneous electronic endoscope apparatus 100.

As shown in Fig. 4, the endoscope apparatus main body 105 comprises: a light source section 41 that primarily acts as an illuminating section; a control section 42 and a main body processing apparatus 43. The control section 42 and the main body processing apparatus 43 control operations of the light source section 41 and/or a CDD 21 as an image pickup section, and constitute a signal processing control section that outputs an image pickup signal to the display monitor 106 that is a display device.

Incidentally, for the present example, while a description will be given on the assumption that the light source section 41 and the main body processing apparatus 43 that performs image processing and the like are provided within the endoscope apparatus main body 105 that is a single unit, the light source section 41 and the main body processing apparatus 43 may alternatively be configured as a detachable unit that is separate from the endoscope apparatus main body 105.

The light source section 41 is connected to the control section 42 and the endoscope 101. The light source section 41 irradiates a white light (including light that is not perfectly white) at a predetermined light quantity based on a signal from the control section 42. In addition, the light source section 41 comprises: a lamp 15 as a white light source; a chopper 16 as a light quantity control section; and a chopper driving section 17 for driving the chopper 16.

As shown in Fig. 5, the chopper 16 is configured as a disk-like structure having a predetermined radius r around a central point 17a and having notched portions of predetermined circumferential lengths. The central point 17a is connected to a rotary shaft provided at the chopper driving section 17. In other words, the chopper 16 performs rotational movement around the central point 17a. In addition, a plurality of notched portions are provided in intervals of a predetermined radius. In the diagram, from radius r0 to radius ra, the notched portion has a maximum length of $2\pi r \times \theta 0$ degrees/360 degrees and a width of r0-ra. In a similar manner, the notched portion is configured so as to have, from radius ra to radius rb, a maximum length of $2\pi ra \times 2\theta 1$ degrees/360 degrees and a width of ra-rb, and from radius rb to radius rc, a maximum length of $2\pi rb \times 2\theta 2$ degrees/360 degrees and a width of rb-rc (where the respective radii have a relationship of r0>ra>rb>rc).

The lengths and widths of the notched portions of the chopper 16 are merely exemplary and are not limited to the present example.

In addition, the chopper 16 has a protruding portion 160a that radially extends at an approximate center of the notched portion. The control section 42 is arranged so as to minimize intervals of light irradiated before and after 1 frame to minimize blurring due to the movement of the subject to be examined by switching frames when light is cut off by the protruding portion 160a.

Furthermore, the chopper driving section 17 is configured so as to be movable in a direction facing the lamp 15 as is indicated by the arrow in Fig. 4.

In other words, the control section 42 is able to change a direction R between the rotational center 17a of the chopper 16 shown in Fig. 5 and a light flux (indicated by the dotted circle) from the lamp. For example, in the state shown in Fig. 5, since the distance R is considerably small, illumination light quantity is low. By increasing the distance R (moving the chopper driving section 17 away from the lamp 15), the notched portion through which the light flux is passable becomes longer, thereby extending irradiating time and enabling the control section 42 to increase illumination light quantity.

As described above, with the electronic endoscope apparatus, since there is a possibility that the S/N ratio of a newly created spectral image is insufficient and a saturation of any of the necessary RGB signals upon creation of a spectral image results in improper computation, it is necessary to control illumination light quantity. The chopper 16 and the chopper driving section 17 are responsible for light quantity adjustment.

In addition, the endoscope 101 connected to the light source section 41 via the connector 11 comprises: an objective lens 19 on the distal end portion 103; and a solid state image pickup device 21 such as a CCD or the like (hereinafter simply referred to as CCD). The CCD 21 constitutes an image pickup section that photoelectrically converts light reflected from a living body that is a subject to be examined based on the irradiating light from the light source section 41 constituting an illumination section and creates an image pickup signal. The CCD in the present example is of the single-plate type (the CCD used in a synchronous electronic endoscope), and is of the primary color-type. Fig. 6 shows an array of color filters positioned on an image pickup plane of the CCD. In addition, Fig. 7 shows respective spectral sensitivity characteristics of RGB of the color filters shown in Fig. 6.

Furthermore, as shown in Fig. 4, the insertion portion 102 comprises: a light guide 14 that guides light irradiated from the light source section 41 to the distal end portion 103; a signal line for transferring an image of the subject to be examined obtained by the CCD to the main body processing apparatus 43; and a forceps channel 28 or the like for performing treatment. Incidentally, a forceps aperture 29 for inserting forceps into the forceps channel 28 is provided in the vicinity of an operating section 104.

Moreover, in the same manner as the light source section 41, the main body processing apparatus 43 is connected to the endoscope 101 via the connector 11. The main body processing apparatus 43 is provided with a CCD driving circuit 431 for driving the CCD 21. In addition, the main body processing apparatus 43 is provided with a luminance signal processing system and a color signal processing system as signal circuit systems for obtaining a normal image.

The luminance signal processing system comprises: a contour correcting section 432 connected to the CCD 21 and which performs contour correction; and a luminance signal processing section 434 that creates a luminance signal from data corrected by the contour correcting section 432. In addition, the color signal processing system comprises: sample-and-hold circuits (S/H circuits) 433a to 433c, connected to the CCD 21, which perform sampling and the like on a signal obtained by the CCD 21 and create an RGB signal; and a color signal processing section 435 connected to outputs of the S/H circuits 433a to 433c and which creates color signals.

Furthermore, a normal image creating section 437 that creates a single normal image from outputs of the luminance signal processing system and the color signal processing system is provided, whereby a Y signal, an R-Y signal and a B-Y signal are sent from the normal image creating section 437 to the display monitor 106 via the switching section 439.

On the other hand, a matrix computing section 436 that receives input of output (RGB signals) of the S/H circuits 433a to 433c and performs predetermined matrix computation on the RGB signals is provided as a signal circuit system for obtaining spectral images. Matrix computation refers to addition processing of color image signals and to processing of multiplying the matrix obtained by the above-described matrix calculating method (or modification thereof).

In the present embodiment, while a method using electronic circuit processing (processing by hardware using an electronic circuit) will be described as the matrix calculating method, a method using numerical data processing (processing by software using a program) such as in an example described later may be used instead. In addition, upon execution, a combination of the methods may also be used.

Fig. 8 is a circuit diagram of the matrix computing section 436. RGB signals are respectively inputted to amplifiers 32a to 32c via resistor groups 31 a to 31 c. The respective resistor groups have a plurality of resistors to which RGB signals are respectively connected, and the resistance values of the respective resistors are values corresponding to the matrix coefficient. In other words, the gain of the RGB signals are varied by the respective resistors and added (or subtracted) by the amplifiers. The respective outputs of the amplifiers 32a to 32c become outputs of the matrix computing section 436. In other words, the matrix computing section 436 performs so-called weighting addition processing. Incidentally, the resistance values of the respective resistors used herein may be arranged to be variable.

An output of the matrix computing section 436 is inputted to the integrating sections 438a to 438c, respectively, to be subjected to integral computation. Subsequently, color adjustment computation to be described later is performed at the color adjusting section 440 on respective spectral image signals $\sum F1$ to $\sum F3$ of the integrating sections, and color channels Rch, Gch and Bch are created from the spectral image signals $\sum F1$ to $\sum F3$. The created color channels Rch, Gch and Bch are sent to the display monitor 106 via a switching section 439. A configuration of the color adjusting section 440 shall be described later.

Incidentally, the switching section 439 is provided for switching between a normal image and a spectral image, and is also capable of switching/displaying among spectral images. In other words, the operator can cause an image among a normal image, an Rch spectral channel image, a Gch spectral channel image and a Bch spectral channel image, to be selectively displayed on the display monitor 106. Furthermore, it may also be configured so that any two or more images are simultaneously displayable on the display monitor 106. In particular, in the case where a normal image and a spectral channel image are simultaneously displayable, it is able to readily compare a spectral channel image against a generally observed normal image. Moreover, the user is able to perform observation of normal images and spectral channel images while taking into consideration the respective features thereof (a feature of normal images is that the color tones thereof closely resemble that of naked eye observation for easy observation; a feature of spectral channel images is that observation of predetermined blood vessels or the like which cannot be observed through normal images are possible), and is extremely useful in diagnostics.

Next, a detailed description on operations of the electronic endoscope apparatus 100 according to the present example will be given with reference to Fig. 4.

In the following, operations during normal image observation will be described first, followed by a description on operations during spectral image observation.

First, to describe operations of the light source section 41, based on a control signal from the control section 42, the chopper driving section 17 is set to a predetermined position and rotates the chopper 16. A light flux from the lamp 15 passes through a notched portion of the chopper 16, and is collected by a collecting lens at an incident end of the light guide 14 that is a light fiber bundle provided inside the connector 11 located at a connecting portion of the endoscope 101 and the light source section 41.

The collected light flux passes the light guide 14 and is irradiated into the body of a subject to be examined from an illuminating optical system provided at the distal end portion 103. The irradiated light flux is reflected inside the subject to be examined, and signals are collected via the objective lens 19 by the CCD 21 according to each color filter shown in Fig. 6.

The collected signals are inputted in parallel to the luminance signal processing system and the color signal processing system described above. Signals collected according to color filter are added on a per-pixel basis and inputted to the contour correcting section 432 of the luminance signal system, and after contour correction, inputted to the luminance signal processing section 434. A luminance signal is created at the luminance signal processing section 434, and is inputted to the normal image creating section 437.

Meanwhile, signals collected by the CCD 21 is inputted on a per-color filter basis to the S/H circuits 433a to 433c, and R/GB signals are respectively created. In addition, after the R/GB signals are subjected to color signal processing at the color signal processing section 435, a Y signal, an R-Y signal and a B-Y signal are created at the normal image creating section 437 from the afore-mentioned luminance signals and color signals, via the switching section 439, a normal image of the subject to be examined is displayed on the display monitor 106.

Next, operations during spectral image observation will be described. Incidentally, descriptions on operations similar to those performed during normal image observation shall be omitted.

The operator issues an instruction for observing a spectral image from a normal image by operating a keyboard provided on the endoscope apparatus main body 105, a switch provided on the operating section 104 of the endoscope 101, or the like. At this point, the control section 42 changes the control state of the light source section 41 and the main body processing apparatus 43.

More specifically, as required, the light quantity irradiated from the light source section 41 is changed. As described above, since saturation of an output from the CCD 21 is undesirable, during spectral image observation, it reduces illumination light quantity in comparison to normal image observation. Furthermore, in addition to controlling light quantity so that an output signal from the CCD does not reach saturation, the control section 42 is also able to change illumination light quantity within a range in which saturation is not reached.

In addition, as for changing control over the main body processing apparatus 43 by the control section 42, a signal outputted from the switching section 439 is switched from an output of the normal image creating section 437 to an output of the color adjusting section 440. In addition, the outputs of the S/H circuits 433a to 433c are subjected to amplification/addition processing at the matrix computing section 436, outputted according to each band to the integrating sections 438a to 438c, and after integration processing, outputted to the color adjusting section 440. Even when illumination light quantity is reduced by the chopper 16, storage and integration by the integrating sections 438a to 438c enable signal intensity to be increased as shown in Fig. 2, and a spectral image with improved S/N ratio can be obtained.

A specific description will now be given on matrix processing performed by the matrix computing section 436 according to the present example. In the present example, when attempting to create bandpass filters (hereinafter referred to as a quasi-bandpass filters) closely resembling ideal narrowband pass filters F1 to F3 (in this case, the respective wavelength transmitting ranges are assumed to be F1: 590 nm to 620 nm, F2: 520 nm to 560 nm, and F3: 400 nm to 440 nm) depicted in Fig. 7 from the spectral sensitivity characteristics of the RGB color filters indicated by the solid lines in Fig. 7, according to the contents represented by Formulas 1 to 5 presented above, the following matrix becomes optimum.

$$\mathbf{A} \simeq \begin{pmatrix} 0.625 & -3.907 & -0.05 \\ -3.097 & 0.631 & -1.661 \\ 0.036 & -5.146 & 0.528 \end{pmatrix} \quad \cdots(19)$$

Furthermore, by performing correction using contents represented by Formulas 6 and 7, the following coefficient is obtained.

$$\mathbf{K} = \begin{pmatrix} 1 & 0 & 0 \\ 0 & 1.07 & 0 \\ 0 & 0 & 1.57 \end{pmatrix} \quad \cdots(20)$$

Incidentally, the above uses a priori information that the spectrum $S(\lambda)$ of a light source represented by Formula 6 is depicted in Fig. 9 and the reflectance spectrum $H(\lambda)$ of the living body to be studied represented by Formula 7 is depicted in Fig. 10.

Therefore, the processing performed by the matrix computing section 436 is mathematically equivalent to the matrix computation below.

$$\mathbf{A'} = \mathbf{KA} = \begin{pmatrix} 1 & 0 & 0 \\ 0 & 1.07 & 0 \\ 0 & 0 & 1.57 \end{pmatrix} \begin{pmatrix} 0.625 & -3.907 & -0.05 \\ -3.097 & 0.631 & -1.661 \\ 0.036 & -5.146 & 0.528 \end{pmatrix}$$

$$= \begin{pmatrix} 0.625 & -3.907 & -0.050 \\ -3.314 & 0.675 & -1.777 \\ 0.057 & -8.079 & 0.829 \end{pmatrix} \quad \cdots(21)$$

By performing the matrix computation, quasi-filter characteristics (indicated as characteristics of quasi-filters F1 to F3 in Fig. 7) are obtained. In other words, the aforementioned matrix processing is for creating a spectral image signal by using a quasi-bandpass filter (that is, matrix) created in advance as described above on a color image signal.

An example of an endoscopic image created using the quasi-filter characteristics will be shown below.

As shown in Fig. 11, tissue inside a body cavity 45 often has an absorbing body distributed structure such as blood vessels which differ in a depth direction. Capillaries 46 are predominantly distributed in the vicinity of the surface layers of the mucous membrane, while veins 47 larger than capillaries are distributed together with capillaries in intermediate layers that are deeper than the surface layers, and even larger veins 48 are distributed in further deeper layers.

On the other hand, the reachable depth of light in the depth-wise direction of the tissue inside a body cavity 45 is dependent on the wavelength of the light. As shown in Fig. 12, in the case of a light having a short wavelength such as blue (B), illumination light including the visible range only reaches the vicinity of the surface layers due to absorption characteristics and scattering characteristics of the biological tissue. Thus, the light is subjected to absorption and scattering within a range up to that depth, and light exiting the surface is observed. Furthermore, in the case of green (G) light whose wavelength is longer than that of blue (B) light, light reaches a greater depth than the reachable range of blue (B) light. Thus, light is subjected to absorption and scattering within the range, and light exiting the surface is observed. Moreover, red (R) light whose wavelength is longer than that of green (G) light reaches an even greater depth.

As shown in Fig. 13, with RGB light during normal observation of the tissue inside a body cavity 51, since the respective wavelength band overlap each other:

(1) an image pickup signal picked up by the CCD 21 under B band light picks up a band image having superficial and intermediate tissue information including a large amount of superficial tissue information such as that shown in Fig. 14;

(2) an image pickup signal picked up by the CCD 21 under G band light picks up a band image having superficial and intermediate tissue information including a large amount of intermediate tissue information such as that shown in Fig. 15; and

(3) an image pickup signal picked up by the CCD 21 under R band light picks up a band image having intermediate and deep tissue information including a large amount of deep tissue information such as that shown in Fig. 16.

In addition, by performing signal processing on the RGB image pickup signals at the endoscope apparatus main body 105, it is now possible to obtain a desirable endoscopic image or an endoscopic image with natural color reproduction.

The matrix processing performed by the above-described matrix computing section 436 is for creating a spectral image signal using a quasi-bandpass filter (matrix) created in advance as described above on a color image signal. For example, spectral image signals F1 to F3 are obtained by using quasi-bandpass filters F1 to F3 having discrete narrowband spectral characteristics and which are capable of extracting desired deep tissue information, as shown in Fig. 17. As shown in Fig. 17, since the respective wavelength ranges of the quasi-bandpass filters F1 to F3 do not overlap each other,

(4) a band image having superficial layer tissue information such as that shown in Fig. 18 is picked up in the spectral image signal F3 by the quasi-bandpass filter F3;

(5) a band image having intermediate layer tissue information such as that shown in Fig. 19 is picked up in the spectral image signal F2 by the quasi-bandpass filter F2; and

(6) a band image having deep layer tissue information such as that shown in Fig. 20 is picked up in the spectral image signal F1 by the quasi-bandpass filter F1.

Next, with respect to the spectral image signals F1 to F3 obtained in this manner, as an example of a most simplified color conversion, the color adjusting section 440 respectively allocates the spectral image signal F1 to the color channel Rch, the spectral image signal F2 to the color channel Gch and the spectral image signal F3 to the color channel Bch, and outputs the same via the switching section 439 to the display monitor 106.

As shown in Fig. 21, the color adjusting section 440 is constituted by a color conversion processing circuit 440a comprising: a 3 by 3 matrix circuit 61; three sets of LUTs 62a, 62b, 62c, 63a, 63b and 63c provided anteriorly and posteriorly to the 3 by 3 matrix circuit 61; and a coefficient changing circuit 64 that changes table data of the LUTs 62a, 62b, 62c, 63a, 63b and 63c or the coefficient of the 3 by 3 matrix circuit 61.

The spectral image signals F1 to F3 inputted to the color conversion processing circuit 440a are subjected to inverse $\gamma$ correction, non-linear contrast conversion processing and the like on a per-band data basis by the LUTs 62a, 62b and 62c.

Then, after color conversion is performed at the 3 by 3 matrix circuit 61, $\gamma$ correction or appropriate tone conversion processing is performed at the post-stage LUTs 63a, 63b and 63c.

Table data of the LUTs 62a, 62b, 62c, 63a, 63b and 63c or the matrix coefficient of the 3 by 3 matrix circuit 61 can be changed by the coefficient changing circuit 64.

Changes by the coefficient changing circuit 64 are performed based on a control signal from a processing converting switch (not shown) provided on the operating section of the endoscope 101 or the like.

Upon receiving the control signal, the coefficient changing circuit 64 reads out appropriate data from coefficient data stored in advance in the color adjusting section 440, and overwrites the current circuit coefficient with the data.

Next, specific contents of color conversion processing will be described. Formula 22 represents an example of a color conversion equation.

$$\begin{pmatrix} R_{ch} \\ G_{ch} \\ B_{ch} \end{pmatrix} = \begin{pmatrix} 1 & 0 & 0 \\ 0 & 1 & 0 \\ 0 & 0 & 1 \end{pmatrix} \begin{pmatrix} F_1 \\ F_2 \\ F_3 \end{pmatrix} \quad \cdots (22)$$

The processing represented by Formula 22 is color conversion in which spectral image signals F1 to F3 are assigned to the spectral channel images Rch, Gch and Bch in ascending order of wavelengths.

In a case of observation with a color image based on the color channels Rch, Gch and Bch, for example, an image such as that shown in Fig. 22 is obtained. A large vein exists at a deep position on which the spectral image signal F3 is reflected, and as for color, the large vein is shown as a blue pattern. Since the spectral image signal F2 is strongly reflected on a vascular network near intermediate layers, the vascular network is shown as a color image in a red pattern. Among vascular networks, those existing near the surface of the mucosal membrane are expressed as a yellow pattern.

In particular, changes in the pattern in the vicinity of the surface of the mucosal membrane are important for the discovery and differential diagnosis of early-stage diseases. However, a yellow pattern tends to have a weak contrast against background mucosa and therefore low visibility.

In this light, in order to more clearly reproduce patterns in the vicinity of the surface of the mucosal membrane, a conversion expressed by Formula 23 becomes effective.

$$\begin{pmatrix} R_{ch} \\ G_{ch} \\ B_{ch} \end{pmatrix} = \begin{pmatrix} 1 & 0 & 0 \\ 0 & \omega_G & \omega_B \\ 0 & 0 & 1 \end{pmatrix} \begin{pmatrix} F_1 \\ F_2 \\ F_3 \end{pmatrix} \quad \cdots (23)$$

The processing represented by Formula 23 is an example of a conversion in which the spectral image signal F1 is mixed with the spectral image signal F2 at a certain ratio and created data is newly used as the spectral G channel image Gch, and enables further clarification of the fact that absorbing/scattering bodies such as a vascular network differ according to depth position.

Therefore, by adjusting the matrix coefficient via the coefficient changing circuit 64, the user is able to adjust display colors. As for operations, in conjunction with a mode switching switch (not shown) provided at the operating section of the endoscope 101, the matrix coefficient is set to a default value from a through operation in the color conversion processing circuit 440a.

A through operation in this case refers to a state in which a unit matrix is mounted on the 3 by 3 matrix circuit 61 and a non-conversion table is mounted on the LUTs 62a, 62b, 62c, 63a, 63b and 63c. This means that, for example, preset values of $\omega_G$=0.2, $\omega_B$=0.8 are to be provided as default values of the matrix coefficient $\omega$G, $\omega$B.

Then, by operating the operating section of the endoscope 101 or the like, the user performs adjustment so that the coefficient becomes, for example, $\omega_G$=0.4, $\omega_B$=0.6. An inverse $\gamma$ correction table and a $\gamma$ correction table are applied as required to the LUTs 62a, 62b, 62c, 63a, 63b and 63c.

While the color conversion processing circuit 440a is arranged to perform color conversion by a matrix computing unit constituted by the 3 by 3 matrix circuit 61, the arrangement is not restrictive and, instead, color conversion processing means may be configured using a numerical processor (CPU) or an LUT.

For example, in the above-described example, while the color conversion processing circuit 440a is illustrated by a configuration centered around the 3 by 3 matrix circuit 61, similar advantages may be achieved by replacing the color conversion processing circuit 440a with three-dimensional LUTs 65 corresponding to each band as shown in Fig. 23. In this case, the coefficient changing circuit 64 performs an operation for changing the table contents based on a control signal from a processing converting switch (not shown) provided on the operating section of the endoscope 101 or the like.

Incidentally, the filter characteristics of the quasi-bandpass filters F1 to F3 are not limited to the visible range. As a first modification of the quasi-bandpass filters F1 to F3, filter characteristics may be arranged as, for example, a narrowband having discrete spectral characteristics such as those shown in Fig. 24. By setting F3 in the near-ultraviolet range and setting F1 in the near-infrared range in order to observe irregularities on a living body surface and absorbing bodies in the vicinity of extremely deep layers, the filter characteristics of the first modification is suitable for obtaining image information unobtainable through normal observation.

In addition, as a second modification of the quasi-bandpass filters F1 to F3, as shown in Fig. 25, the quasi-bandpass filter F2 may be replaced by two quasi-bandpass filters F3a and F3b having adjacent filter characteristics in the short wavelength range. This modification takes advantage of the fact that wavelength ranges in the vicinity thereof only reach the vicinity of the uppermost layers of a living body, and is suitable for visualizing subtle differences in scattering characteristics rather than absorption characteristics. From a medical perspective, utilization in the discriminatory diagnosis of early carcinoma and other diseases accompanied by a disturbance in cellular arrangement in the vicinity of the surface of mucous membrane is envisaged.

Furthermore, as a third modification of the quasi-bandpass filters F1 to F3, as shown in Fig. 26, two quasi-bandpass filters F2 and F3 having dual-narrowband filter characteristics with discrete spectral characteristics and which are capable of extracting desired layer-tissue information can be arranged to be created by the matrix computing section 436.

In the case of the quasi-bandpass filters F2 and F3 shown in Fig. 26, for the colorization of an image during narrowband spectral image observation, the color adjusting section 440 creates color images of the three RGB channels such that: spectral channel image Rch ← spectral image signal F2; spectral channel image Gch ← spectral image signal F3; and spectral channel image Bch ← spectral image signal F3.

In other words, for the spectral image signals F2 and F3, the color adjusting section 440 creates color images (Rch, Gch and Bch) of the three RGB channels from Formula 24 below.

$$\begin{pmatrix} R_{ch} \\ G_{ch} \\ B_{ch} \end{pmatrix} = \begin{pmatrix} h_{11} & h_{12} \\ h_{21} & h_{22} \\ h_{31} & h_{32} \end{pmatrix} \begin{pmatrix} F_2 \\ F_3 \end{pmatrix} \qquad \cdots (24)$$

For example, let us assume that h11 = 1, h12 = 0, h21 = 0, h22 = 1.2, h31 = 0, and h32 = 0.8.

For example, the spectral image F3 is an image whose central wavelength mainly corresponds to 415 nm, and the spectral image F2 is an image whose central wavelength mainly corresponds to 540 nm.

Furthermore, for example, even when computation is performed on the assumption that the spectral image F3 is an image whose central wavelength mainly corresponds to 415 nm, the spectral image F2 is an image whose central wavelength mainly corresponds to 540 nm, and the spectral image F1 is an image whose central wavelength mainly corresponds to 600 nm, a color image may be formed by the color adjusting section 440 from the F2 and F3 images without using the F1 image. In this case, it will suffice to apply a matrix computation expressed by Formula 24' below instead of Formula 24.

$$Rch = h11 \times F1 + h12 \times F2 + h13 \times F3$$

$$Gch = h21 \times F1 + h22 \times F2 + h23 \times F3$$

$$Bch = h31 \times F1 + h32 \times F2 + h33 \times F3 \qquad \cdots (24')$$

In the matrix computation expressed by Formula 24' above, it will suffice to set the coefficients of h11, h13, h21, h22, h31 and h32 to 0 while setting other coefficients to predetermined numerical values.

As seen, according to the present example, by creating a quasi-narrowband filter using a color image signal for creating a normal electronic endoscopic image (normal image), a spectral image having tissue information of a desired depth such as a vascular pattern can be obtained without having to use an optical wavelength narrow bandpass filter for spectral images. Additionally, by setting a parameter of the color conversion processing circuit 440a of the color adjusting section 440 in accordance to the spectral image, it is now possible to realize a representation method that makes full use of a feature that is reachable depth information during narrowband spectral image observation, and consequently, effective separation and visual confirmation of tissue information of a desired depth in the vicinity of the tissue surface of biological tissue can be realized.

Furthermore, in particular, with the color adjusting section 440:

(1) in the case of a two-band spectral image, when an image corresponding to, for example, 415 nm is allocated to the color channels Gch and Bch and an image corresponding to, for example, 540 nm is allocated to the color channel Rch; or

(2) in the case of a three-band spectral image, when an image corresponding to, for example, 415 nm is allocated to the color channel Bch, an image corresponding to, for example, 445 nm is allocated to the color channel Gch and an image corresponding to, for example, 500 nm is allocated to the color channels Rch, the following image effects are achieved.

- High visibility of capillaries in an uppermost layer of a biological tissue is attained by reproducing epithelia or mucosa in an uppermost layer of the biological tissue in a color having low chroma and by reproducing capillaries in the uppermost layer in low luminance or, in other words, as dark lines.
- At the same time, since blood vessels positioned deeper than capillaries are reproduced by rotating towards blue in a hue-wise direction, discrimination from capillaries in the uppermost layer becomes even easier.

Moreover, according the above-described channel allocation method, residue and bile that are observed in a yellow tone under normal observation during endoscopic examination of the large intestine are now observed in a red tone.

Fig. 27 is a block diagram showing another configuration example of the matrix computing section.

Components other than the matrix computing section 436 are the same as those in Fig. 4. The sole difference lies in the configuration of the matrix computing section 436 shown in Fig. 27 from the configuration of the matrix computing section 436 shown in Fig. 8. Only differences will now be described, and like components will be assigned like reference characters and descriptions thereof will be omitted.

While it is assumed in Fig. 8 that matrix computation is performed by so-called hardware processing using an electronic circuit, in Fig. 27, the matrix computation is performed by numerical data processing (processing by software using a program).

The matrix computing section 436 shown in Fig. 27 includes an image memory 50 for storing respective color image signals of R, G and B. In addition, a coefficient register 151 is provided in which respective values of the matrix <A'> expressed by Formula 21 are stored as numerical data.

The coefficient register 51 and the image memory 50 are connected to multipliers 53a to 53i; the multipliers 53a, 52d and 53g are connected in turn to a multiplier 54a; and an output of the multiplier 54a is connected to the integrating section 438a shown in Fig. 4. In addition, the multipliers 53b, 52e and 53h are connected to a multiplier 54b, and an output thereof is connected to the integrating section 438b. Furthermore, the multipliers 53c, 52f and 53i are connected to a multiplier 54c, and an output thereof is connected to the integrating section 438c.

As for operations in the present example, inputted RGB image data is temporarily stored in the image memory 50. Next, a computing program stored in a predetermined storage device (not shown) causes each coefficient of the matrix <A'> from the coefficient register 51 to be multiplied at a multiplier by RGB image data stored in the image memory 50.

Incidentally, Fig, 27 shows an example in which the R signal is multiplied by the respective matrix coefficients at the multipliers 53a to 53c. In addition, as is shown in the same diagram, the G signal is multiplied by the respective matrix coefficients at the multipliers 53d to 53f, while the B signal is multiplied by the respective matrix coefficients at the multipliers 53g to 53i. As for data respectively multiplied by the matrix coefficients, outputs of the multipliers 53a, 52d and 53g are multiplied by the multiplier 54a, outputs of the multipliers 53b, 52e and 53h are multiplied by the multiplier 54d, and the outputs of the multipliers 53c, 52f and 53i are multiplied by the multiplier 54c. An output of the multiplier 54a is sent to the integrating section 438a. In addition, the outputs of the multipliers 53b and 53c are respectively sent to the integrating sections 438b and 438c.

According to the configuration example shown in Fig. 27, in the same manner as the configuration example shown in Fig. 8, a spectral image on which vascular patterns are clearly displayed can be obtained.

Moreover, with the configuration example shown in Fig. 27, since matrix processing is performed using software without using hardware as is the case with the configuration example shown in Fig. 8, for example, changes to each matrix coefficient or the like can be accommodated in a prompt manner.

In addition, in a case where matrix coefficients are stored by resultant values alone or, in other words, not stored as a matrix <A'> but stored instead according to $S(\lambda)$, $H(\lambda)$, $R(\lambda)$, $G(\lambda)$ and $B(\lambda)$, and computed as required to determine a matrix <A'> for subsequent use, it is possible to change just one of the elements, thereby improving convenience. For example, it is possible to change only the illumination light spectral characteristics $S(\lambda)$ or the like.

[Second example]

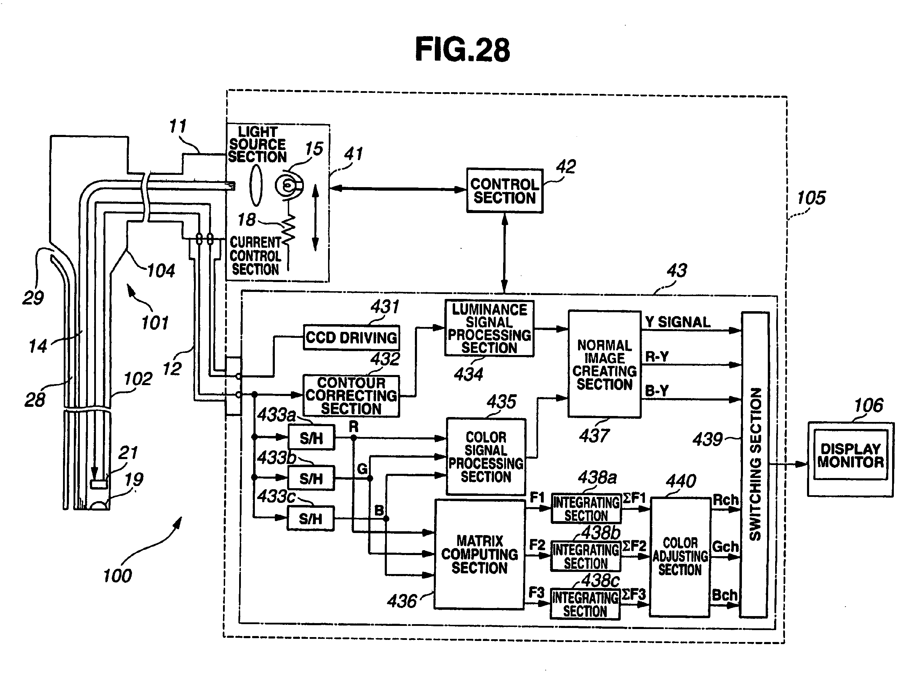

Fig. 28 is a block diagram showing a configuration of an electronic endoscope apparatus according to a second example useful for understanding the present invention.

Since the second example is practically the same as the first example, only differences therefrom will be described. Like components will be assigned like reference characters and descriptions thereof will be omitted.

The present example differs from the first example in the light source section 41 that performs illumination light quantity control. In the present example, control of light quantity irradiated from the light source section 41 is performed by controlling the current of the lamp 15 instead of by a chopper. More specifically, a current control section 18 as a light quantity control section is provided at the lamp 15 shown in Fig. 28.

As for operations of the present example, the control section 42 controls the current flowing through the lamp 51 so that neither of the color image signals of RGB reach a saturated state. Consequently, since the current used by the lamp 15 for emission is controlled, the light quantity thereof varies according to the magnitude of the current.

Incidentally, since other operations are the same as those in the first example, descriptions thereof will be omitted.

According to the present example, in the same manner as the first example, a spectral image on which vascular patterns are clearly displayed can be obtained. In addition, the present example is advantageous in that the control method thereof is simpler than the light quantity control method using a chopper as is the case in the first example.

[Third example]

The biological observation apparatus shown in Fig. 4 performs control during spectral image acquisition so as to reduce light quantity using the chopper 16 shown in Fig. 5 which performs light quantity control by cutting off light at predetermined time intervals. In other words, the light quantity from the light source is reduced so that all color-separated signals of R, G and B are photographed at a suitable dynamic range.

For the third example useful for understanding the present invention, an example will be described in which a movable cutoff member such as a diaphragm spring or a shutter or a cutoff filter such as a mesh turret or an ND filter is used in place of the chopper 16 in the biological observation apparatus shown in Fig. 4.

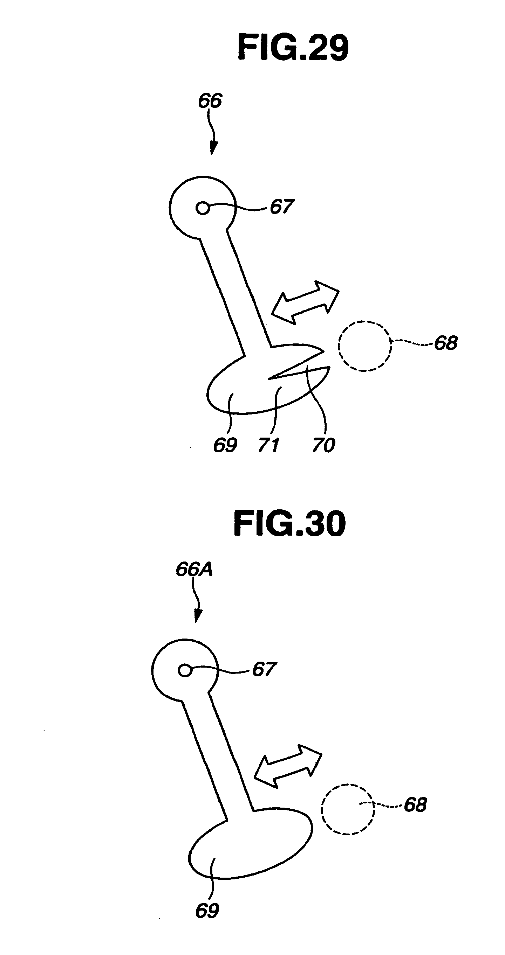

Fig. 29 shows an example of a diaphragm spring 66. The diaphragm spring 66 performs light quantity control by cutting off light at predetermined time intervals using: a cutoff section 69 that rotates around a central axis 67 and which cuts off a light flux 68 converged to a given magnitude at a distal end portion thereof; and a diaphragm blade section 71 having a notched portion 70 that controls output light quantity.

The diaphragm spring 66 may double as a modulating diaphragm spring that controls output light quantity of the light source section 41, or another unit may be separately provided as a cutoff mechanism.

Fig. 30 shows an example of a shutter 66A. While the shutter 66A is similar in shape to the example of the diaphragm spring 66, the structure thereof is such that the notched portion 70 of the diaphragm spring 66 is absent from the cutoff section 69. As for operations of the shutter 66A, light is cut off at predetermined time intervals to perform light quantity control by controlling two operating states of fully open and fully closed.

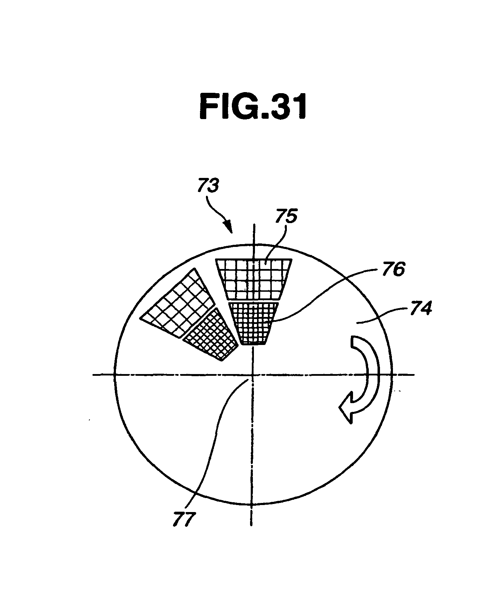

Fig. 31 shows an example of a mesh turret 73. A mesh 75 having wide grid spacing or a mesh 76 with narrower grid spacing is attached by welding or the like to a hole provided on a rotating plate 74, and rotates around a rotation central axis 77. In this case, light is cut off at predetermined time intervals to perform light quantity control by altering mesh length, mesh coarseness, position or the like.

[Embodiment of the invention, fourth example]

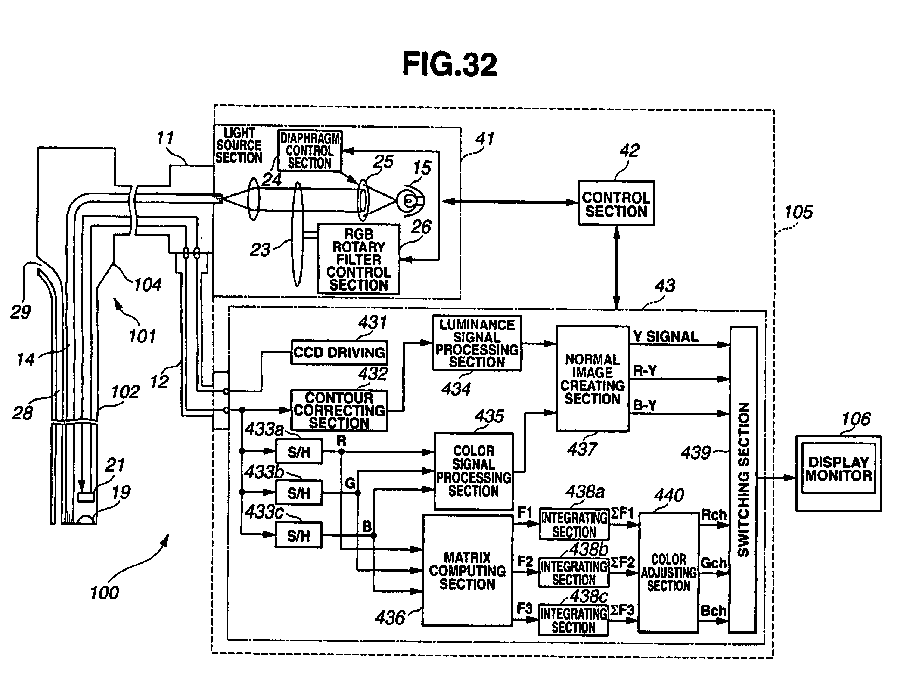

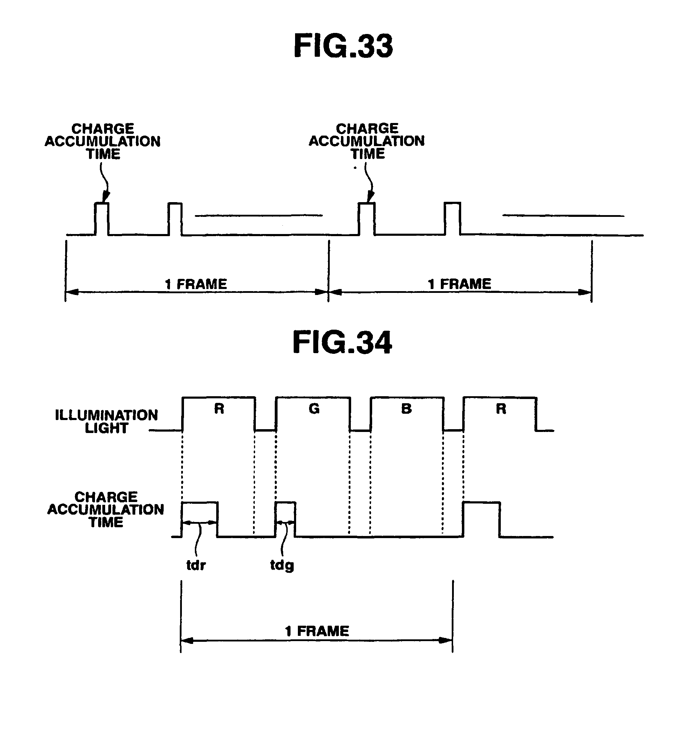

Figs. 32 and 33 relate to a fourth example, which is an embodiment of the present invention, wherein: Fig. 32 is a block diagram showing a configuration of an electronic endoscope apparatus; and Fig. 33 is a diagram showing charge accumulation times of the CCD 21 shown in Fig. 32.

Since the fourth example is practically the same as the first example, only differences therefrom will be described. Like components will be assigned like reference characters and descriptions thereof will be omitted.

The present example primarily differs from the first example in the light source section 41 and the CCD 21. In the first example, the CCD 21 is provided with the color filters shown in Fig. 6 and is a so-called synchronous-type CCD that creates a color signal using the color filters. In contrast thereto, in the present example, a so-called frame sequential-type is used which creates a color signal by illuminating illumination light in the order of R, G and B within a time period of a single frame.

As shown in Fig. 32, the light source section 41 according to the present example is provided with a diaphragm 25 that performs modulation on a front face of the lamp 15, and an RGB rotary filter 23 that makes, for example, one rotation during one frame is further provided on a front face of the diaphragm 25 in order to irradiate R, G and B frame sequential light. In addition, the diaphragm 25 is connected to a diaphragm control section 24 as a light quantity control section, and is arranged so as to be capable of performing modulation by limiting a light flux to be transmitted among light flux irradiated from the lamp 15 to change light quantity in response to a control signal from the diaphragm control section 24. Furthermore, the RGB rotary filter 23 is connected to an RGB rotary filter control section 26 and is rotated at a predetermined rotation speed.

As for operations by the light source section according to the present example, a light flux outputted from the lamp 15 is limited to a predetermined light quantity by the diaphragm 25. The light flux transmitted through the diaphragm 25 passes through the RGB rotary filter 23, and is outputted as respective illumination lights of R/G/B at predetermined time intervals from the light source section. In addition, the respective illumination lights are reflected inside the subject to be examined and received by the CCD 21. Signals obtained at the CCD 21 are sorted according to irradiation time by a switching section (not shown) provided at the endoscope apparatus main body 105, and are respectively inputted to the S/H circuits 433a to 433c. In other words, when an illumination light is irradiated via the R filter from the light source section 41, a signal obtained by the CCD 21 is inputted to the S/H circuit 433a. Incidentally, since other operations are the same as those in the first example, descriptions thereof will be omitted.

According to the present fourth example, in the same manner as the first example, a spectral image on which vascular patterns are clearly displayed can be obtained. In addition, unlike the first example, the present fourth example is able to receive the full benefits of the so-called frame sequential method. Such benefits include, for example, those offered by a modification shown in Fig. 34 which will be described later.

Furthermore, in the first example described above, illumination light quantity (light quantity from a light source) is controlled/adjusted in order to avoid saturation of R/G/B color signals. In contrast thereto, the present fourth example employs a method in which an electronic shutter of the CCD 21 is adjusted. At the CCD 21, charges accumulate in proportion to light intensity incident within a given time period, whereby the charge quantity is taken as a signal. What corresponds to the accumulation time is a so-called electronic shutter. By adjusting the electronic shutter by the CCD driving circuit 431, a charge accumulated quantity or, in other words, a signal quantity can be adjusted. As shown in Fig. 33, by obtaining RGB color images in a state where charge accumulation times are sequentially changed per one frame, a similar spectral image can be obtained. In other words, in each of the examples described above, illumination light quantity control by the diaphragm 25 may be used to obtain a normal image, and when obtaining a spectral image, it is possible to prevent saturation of R, G and B color images by varying the electronic shutter.

Fig. 34 is a diagram showing charge accumulation times of a CCD according to another example of the fourth embodiment of the present invention. The present example is similar to the example shown in Fig. 33 in the utilization of a frame sequential method, and takes advantage of features of the frame sequential method. In other words, by adding weighting respectively for R, G and B to charge accumulation times due to electronic shutter control according to the example shown in Fig. 33, creation of spectral image data can be simplified. This means that, in the example shown in Fig. 34, a CCD driving circuit 431 is provided which is capable of varying the charge accumulation time of the CCD 21 for R, G and B respectively within one frame time period. Otherwise, the present example is the same as the example shown in Fig. 33.

As for operations of the example shown in Fig. 34, when respective illumination lights are irradiated via the RGB rotary filter 23, the charge accumulation time due to the electronic shutter of the CCD 21 is varied.

At this point, let us assume that the respective charge accumulation times of the CCD 21 for R/GB illumination lights are tdr, tdg and tdb (incidentally, since an accumulation time is not provided for the B color image signal, tdb is omitted in the diagram). For example, when performing the matrix computation represented by Formula 21, since the computation to be performed by the F3 quasi-filter image may be determined from RGB images obtained by a normal endoscope as

$$F3 = -0.050R - 1.777G + 0.829B \quad \cdots (25)$$

setting the charge accumulation time due to electronic shutter control according to RGB shown in Fig. 33 to

$$tdr : tdg : tdb = 0.050 : 1.777 : 0.829 \quad \cdots (26)$$

shall suffice. In addition, for the matrix portion, a signal in which only the R and G components are inverted as well as the B component are added. As a result, a spectral image similar to that in the first to third embodiments can be obtained.

According to the fourth example shown in Figs. 33 and 34, a spectral image on which vascular patterns are clearly displayed can be obtained. Furthermore, the example shown in Fig. 34 utilizes the frame sequential method for creating color image signals, and charge accumulation times can be varied using the electronic shutter for each color signal. Consequently, the matrix computing section need only perform addition and subtraction processing, thereby enabling simplification of processing. In other words, operations corresponding to matrix computation may be performed through electronic shutter control, and processing can be simplified.

It is needless to say that the light quantity control of the first to third examples and the electronic shutter (charge accumulation time) control of the fourth example (the example shown in Fig. 33 or 34) can be configured to be performed simultaneously. In addition, as described above, it is obvious that illumination light control may be performed using a chopper or the like for a normal observation image, and when obtaining a spectral observation image, control by an electronic shutter may be performed.

Next, as fifth to seventh examples, a signal amplifying section that amplifies a signal level of an image pickup signal of a normal image and/or a spectral signal of a spectral image, as well as amplification control thereof, will be described.

[Embodiment of the invention, fifth example]

As for a configuration of a biological observation apparatus according to the fifth example, which is an embodiment of the present invention, Fig. 4, 28 or 32 is applied. In addition, AGC (automatic gain control) in the configurations during normal image observation is performed at an AGC circuit (not shown) that is a signal amplifying section for the luminance signal processing section 434 and the color signal processing section 435, respectively, shown in Fig. 4, 28 or 32. AGC during spectral image observation is performed at an AGC circuit (in which, for example, the amplifiers 32a to 32c shown in Fig. 8 are replaced with variable amplifiers) that is a signal amplifying section in the matrix computing section 436 according to Fig. 4, 28 or 32.

Furthermore, control of amplifying operations or, in other words, AGC control is altered between normal image observation and spectral image observation. AGC control refers to an amplification level, an operating speed (follow-up speed), or activation/non-activation (which may also be referred to as on/off) of an amplifying function.

As for the activation/non-activation of the amplifying function, in many cases, AGC is not activated during normal image observation. This is due to the fact that there is sufficient light quantity during observation under a normal light. On the other hand, AGC is activated during spectral image observation since light quantity is insufficient.

As for the operating speed (follow-up speed) of the amplifying function, for example, as a camera moves away from a scene assumed to be a subject, the light quantity gradually decreases and becomes darker. Although a modulating function initially becomes active and attempts to increase light quantity as it becomes dark, the modulating function is unable to follow up. Once follow-up becomes inoperable, AGC is activated. Speed of the AGC operation is important, and an excessive follow-up speed results in an occurrence of noise when dark, which can be annoying. Accordingly, an appropriate speed that is neither too fast nor too slow is imperative. While an AGC operation during normal image observation can afford to be considerably slow, an AGC operation during spectral image observation must be performed at a faster pace due to faster dimming. Consequently, an image quality of a signal to be displayed/outputted can be improved.

[Sixth example]

As for a configuration of a biological observation apparatus according to the sixth embodiment of the present invention, Fig. 4, 28 or 32 is applied. In addition, AGC (automatic gain control) in the configurations during normal image observation is performed at an AGC circuit (not shown) that is a signal amplifying section for the luminance signal processing section 434 and the color signal processing section 435 respectively, shown in Fig. 4, 28 or 32. AGC during spectral image observation is performed at an AGC circuit (in which, for example, the amplifiers 32a to 32c shown in Fig. 8 are replaced with variable amplifiers) that is a signal amplifying section in the matrix computing section 436 according to Fig. 4, 28 or 32.

In the present sixth example, the AGC circuit that is a signal amplifying section is controlled so as to operate in conjunction with a light quantity control section that includes the chopper 16, the lamp current control section 18 or the diaphragm control section 24 and the like. Control of the conjunctional operation described above is performed so that, for example, the AGC circuit that is a signal amplifying section only functions after irradiating light quantity reaches maximum at the light quantity control section. In other words, control is performed so that AGC is activated only after the light quantity control section is controlled to maximum light quantity (when, for example, a modulating blade is fully opened) and when the screen is dark even at the maximum light quantity. Consequently, a range of light quantity control can be expanded.

[Seventh example]

As for a configuration of a biological observation apparatus according to the seventh example of the present invention, Fig. 4, 28 or 32 is applied. In addition, AGC (automatic gain control) in the configurations during normal image observation is performed at an AGC circuit (not shown) that is a signal amplifying section for the luminance signal processing section 434 and the color signal processing section 435, respectively, shown in Fig. 4, 28 or 32. AGC during spectral image observation is performed at an AGC circuit (in which, for example, the amplifiers 32a to 32c shown in Fig. 8 are replaced with variable amplifiers) that is a signal amplifying section in the matrix computing section 436 according to Fig. 4, 28 or 32.

In the event that a normal image and a spectral image are displayed simultaneously (simultaneous display is also possible since a spectral image can be estimated from RGB), there are cases where light quantity is reduced in consideration of CCD saturation. For example, a normal image may have its light quantity reduced in order to suppress CCD saturation. In this case, the normal image is obviously dark. On the other hand, as for a spectral image, adjustment is performed within an appropriate dynamic range so as to allow observation of detailed portions. Therefore, when a normal image and a spectral image are simultaneously displayed without modification means, the normal image remains dark, therefore the brightness of the normal image is adjusted to be increased and outputted to accommodate simultaneous display. Amplification of an image output is performed by electrically increasing gain at the AGC circuit that is a signal amplifying section. Consequently, image quality during simultaneous display can be improved.

Next, image quality improvement will be described with reference to eighth to eleventh examples.

[Eighth example]

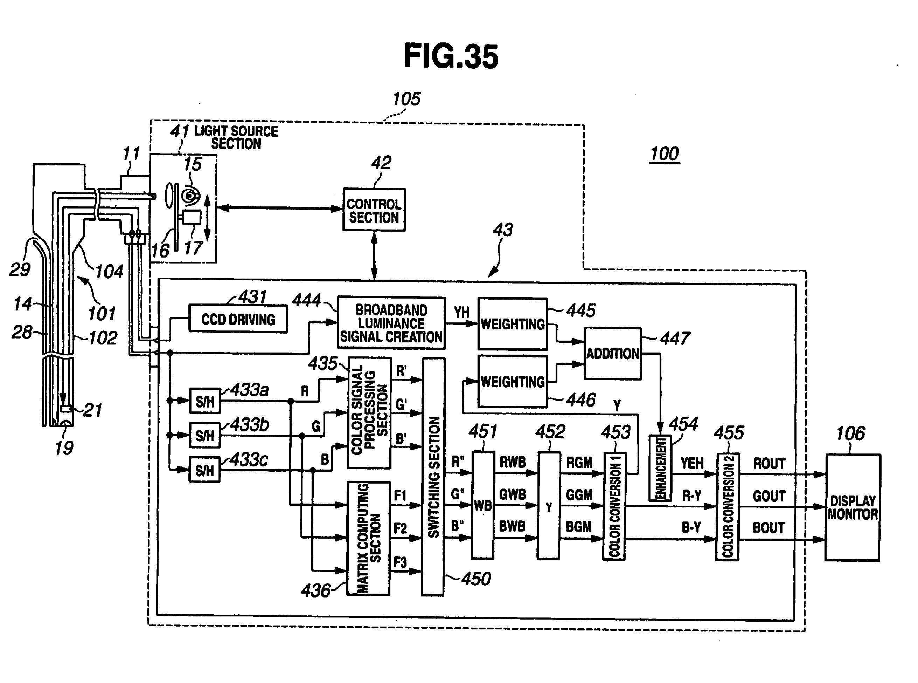

As for a configuration of a biological observation apparatus according to the eighth example of the present invention, Fig. 35 is applied. The present eighth example is intended by reforming weighting addition of a broadband luminance signal to a luminance component of a spectral image to improve brightness and S/N ratio.

In Fig. 35, an electronic endoscope apparatus 100 comprises an electronic endoscope 101, an endoscope apparatus main body 105, and a display monitor 106. The endoscope apparatus main body 105 primarily comprises a light source unit 41, a control section 42, and a main body processing apparatus 43. The main body processing apparatus 43 is provided with a CCD driving circuit 431 for driving the CCD 21, and is also provided with a signal circuit system for obtaining normal images and a signal circuit system for obtaining spectral images.

The signal circuit system for obtaining normal images comprises: S/H circuits 433a to 433c that perform sampling or the like of signals obtained by the CCD 21 and which create an RGB signal; and a color signal processing section 435 connected to outputs of the S/H circuits 433a to 433c and which creates color signals.

On the other hand, a matrix computing section 436 is provided as a signal circuit system for obtaining spectral images at the outputs of the S/H circuits 43 3 a to 43 3 c, whereby a predetermined matrix computation is performed on the RGB signals.

An output of the color signal processing section 435 and an output of the matrix computing section 436 are supplied via a switching section 450 to a white balance processing (hereinafter WB) circuit 451, a γ correcting circuit 452 and a color converting circuit (1) 453 to create a Y signal, an R-Y signal and a B-Y signal. Then, an enhanced luminance signal YEH, an R-Y signal and a B-Y signal to be described later are further created and supplied to a color converting circuit (2) 455, and sent as R, G and B outputs to the display monitor 106.

Incidentally, when conducting spectral image observation (NBI observation) without having an optical filter, a processing system inside the main body processing apparatus (processor) 43 requires a matrix computing section 436 that individually creates spectral images separate from that which creates normal observation images. However, such a configuration in which normal observation images are created separately from spectral images necessitates two separate systems that include white balance processing (WB), γ correcting and color converting circuits, causing an increase in circuit size.

In addition, since an S/N ratio of a spectral image deteriorates when electrically increasing gain in order to enhance brightness, methods for enhancing S/N ratio by picking up and integrating a plurality of images and increasing signal components (for example, integrating sections 438a to 438c in Japanese Patent Laid-Open 2003-93336 correspond to such a method) are proposed. However, obtaining a plurality of images requires a CCD to be driven at a high frequency and is therefore technically difficult.

Thus, in order to solve the above problem, the following configurations are added to the eighth example useful for understanding the present invention as shown in Fig. 35.

Namely,

(1) The following circuits a) to c) are configured to be shared when creating normal observation images and spectral images. a) WB circuit 451, b) γ correcting circuit 452, c) enhancing circuit 454.

Incidentally, circuit sharing is described separately in thirteenth to fifteenth examples.

(2) In order to enhance brightness and S/N ratio, a broadband luminance signal creating section 444 is provided to create a broadband luminance signal (YH) whose S/N ratio has not deteriorated from a CCD output signal, and weighting addition with a luminance component Y of a spectral signal is performed.

More specifically, with respect to the above-mentioned broadband luminance signal (YH) and a luminance signal (Y) of spectral signals (F1, F2 and F3) created at the color converting circuit (1) 453, weighting is respectively performed at weighting circuits (445 and 446), addition is performed at an adding section 447, and contour correction is performed on a post-addition luminance signal at the enhancing circuit 454. In other words, the broadband luminance signal creating section 444, the weighting circuits 445 and 446, and the adding section 447 constitute an image quality adjusting section. A contour-corrected luminance signal YEH is supplied to the color converting circuit (2) 455, and subsequently, once again converted into RGB by the color converting circuit (2) 455 and outputted to the display monitor 106.

Weighting coefficients of the above-described weighting circuits (445 and 446) can be switched according to observation mode or according to a number of pixels of a CCD to be connected thereto, and can be set arbitrarily within such range that does not pose a problem in terms of contrast degradation of a spectral image. For example, when a weighting coefficient of the weighting circuit 445 is denoted by α and a weighting coefficient of the weighting circuit 446 is denoted by β, the following method is conceivable.

A) During display of a normal observation image: α = 0, β = 1
B) During display of a spectral image when a type A CCD is connected: α = 0.5, β = 0.5
C) During display of a spectral image when a type B CCD is connected: α = 1, β = 0

The configuration of the present eighth example is advantageous in that enhancing brightness and S/N ratio is now possible without having to acquire a plurality of images; and since weighting coefficients can be optimized according to type of connected CCDs, optimization according to the number of pixels or to spectral characteristics of each CCD is now possible within such range that does not pose a problem in terms of contrast degradation.

[Ninth example]

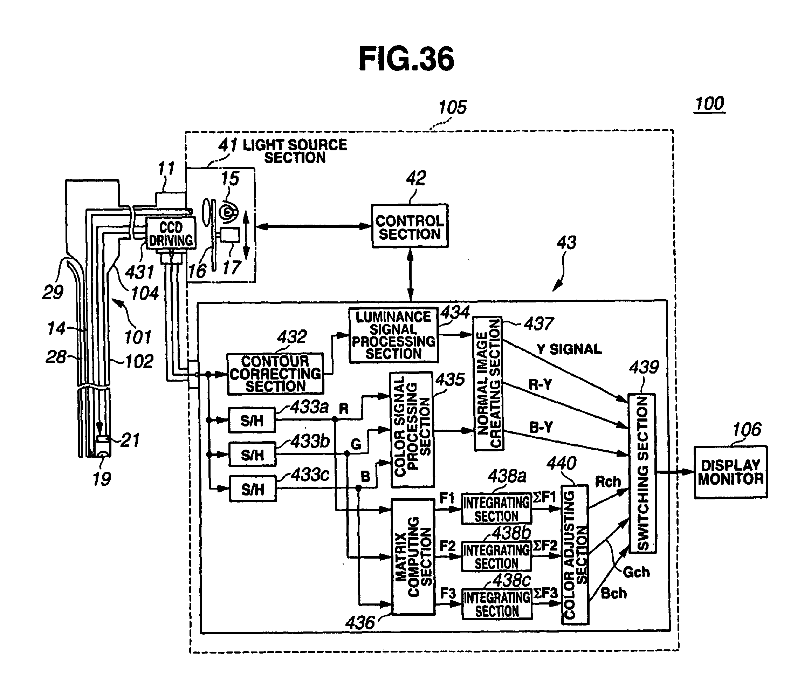

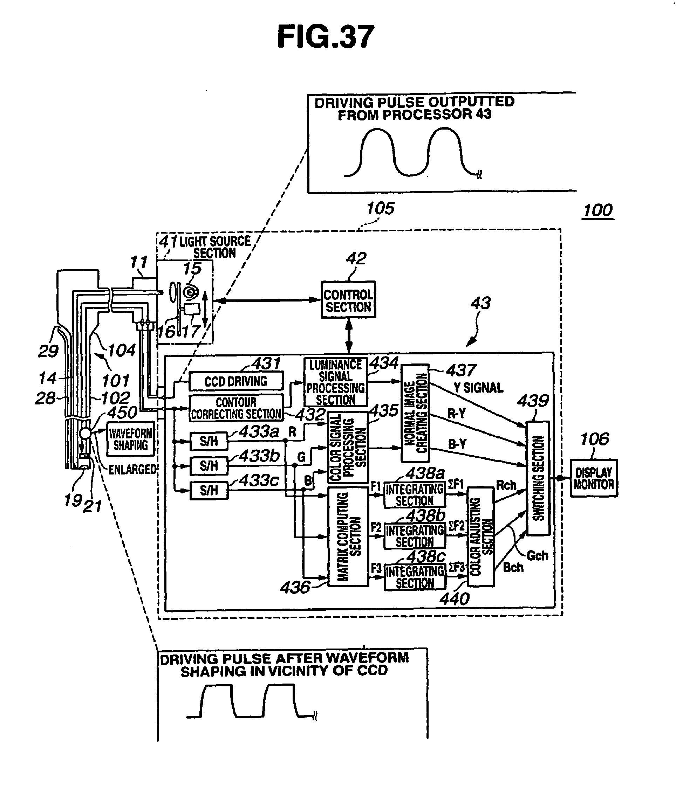

As for a configuration of a biological observation apparatus according to the ninth example useful for understanding the present invention, Fig. 36 or 37 is applied. The present ninth example is arranged to improve S/N ratio.

With the present S/N improvement method, as shown in Fig. 2, illumination light is irradiated in several stages (e.g., n-stages, where n is an integer equal to or greater than 2) within 1 field (1 frame) of a normal image (an ordinary color image) (irradiation intensity may be varied for each stage; in Fig. 2, the stages are denoted by reference characters I0 to In; this procedure can be achieved wholly by controlling illumination light). Consequently, an illumination intensity for each stage can be reduced, thereby enabling suppression of occurrences of saturated states in the respective R, G and B signals. Furthermore, image signals separated into several stages (e.g., n-stages) are subjected to addition corresponding to the number n of image signals at a post-stage. As a result, signal components can be increased to enhance S/N ratio.

As described above, provided is a configuration in which a plurality (n-number) of images is picked up by performing a plurality of image pickups within 1 field time period in order to improve brightness and S/N ratio when conducting NBI observation without having an optical filter, and by adding the plurality of images at a post-stage processing system, signal components can be increased to enhance S/N ratio.

However, the following problems arise when performing a plurality of image pickups within 1 field time period as described in the above configuration.

(1) Since the greater the number of pixels of a CCD, the higher the driving frequency, in a configuration in which the main body processing apparatus (processor) is provided with a driving circuit, a connecting cable to the CCD must be driven by a circuit having high driving performance, thereby presenting a high degree of technical difficulty.
(2) The higher the driving frequency, the higher the frequency of unnecessary radiated electromagnetic field components, thereby making EMC (electromagnetic wave noise) measures difficult.

In order to solve the above problems, the following configurations are added to the ninth example useful for understanding the present invention.

Namely, for example, with respect to the configuration shown in Fig. 4, the CCD driving circuit 431 is relocated from the main body processing apparatus (processor) 43 to the endoscope 101 side as shown in Fig. 36 to realize a configuration in which the length of a connecting cable between the CCD driving circuit 431 and the CCD 21 is minimal.

Consequently, since the cable length is reduced, driving waveform distortion can be reduced. Also, unnecessary EMC radiation is reduced. In addition, since the CCD driving circuit 431 is now on the endoscope 101 side, the driving performance required for the driving circuit can be set low. In other words, a low driving performance is permitted, thereby presenting a cost advantage as well.

Furthermore, for example, with respect to the configuration shown in Fig. 4, while the CCD driving circuit 431 is incorporated in the main body processing apparatus (processor) 43, as shown in Fig. 37, driving pulses are outputted from the main body processing apparatus 43 in a waveform resembling a sinusoidal wave to realize a configuration in which waveform shaping is performed at a waveform shaping circuit 450 provided in the vicinity of the CCD at a distal end of the endoscope 101 to drive the CCD 21.

Consequently, since CCD driving pulses from the main body processing apparatus 43 can be outputted in a waveform resembling a sinusoidal wave, favorable EMC characteristics are attained. In other words, unnecessary radiated electromagnetic fields can be suppressed.

[Tenth example]

As for a configuration of a biological observation apparatus according to the tenth example useful for understanding the present invention, Fig. 4, 28 or 32 is applied. Additionally, in the configurations thereof, a noise suppressing circuit is provided within the matrix computing section 436 required during spectral image observation or an input section at a pre-stage of the matrix computing section 436. Since wavelength band limitation is performed during spectral image observation, a state may occur in which illumination light quantity is lower than during normal image observation. In this case, while a deficiency in brightness due to a low illumination light quantity can be electrically corrected by amplifying a picked up image, simply increasing the gain by an AGC circuit or the like results in an image in which noise is prominent in dark portions thereof. Therefore, by passing image data through the noise suppressing circuit, noise in dark regions are suppressed while contrast degradation in bright regions is reduced. A noise suppressing circuit is described in Fig. 5 of Japanese Patent Application No. 2005-82544.

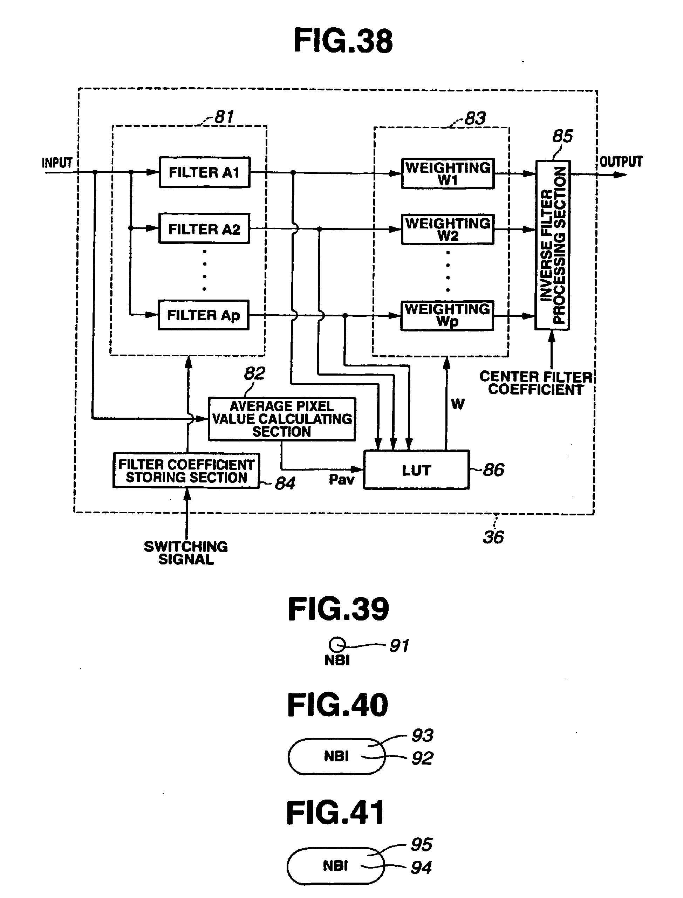

A noise suppressing circuit 36 shown in Fig. 38 is a circuit to be applied to a biological observation apparatus such as that shown in Fig. 32 which handles frame sequential R, G, and B image data. Frame sequential R, G, and B image data is inputted to the noise suppressing circuit.

In Fig. 38, the noise suppressing circuit 36 is configured to comprise: a filtering section 81 that performs filtering using a plurality of spatial filters on image data picked up by a CCD that is image pickup means; an average pixel value calculating section 82 as brightness calculating means that calculates brightness in a localized region of the image data; a weighting section 83 that performs weighting on an output of the filtering section 81 in accordance to the output of the filtering section 81 and/or an output of the average pixel value calculating section 82; and an inverse filter processing section 85 that performs inverse filtering for creating image data subjected to noise suppression processing on an output of the weighting section 83.

p-number of filter coefficients of the filtering section 81 are switched for each R, G, and B input image data, and are read from a filter coefficient storing section 84 and set to respective filters A1 to Ap.

The average pixel value calculating section 82 calculates an average Pav of pixel values of a small region (localized region) of n by n pixels of the same input image data that is used for spatial filtering by the filtering section 81. A weighting coefficient W is read from a look-up table (LUT) 86 according to the average Pav and values of filtering results of the filtering section 81, and set to weighting circuits W1, W2, ..., Wp of the weighting section 83.

According to the circuit shown in Fig. 38, by altering weighting of noise suppression processing by spatial filters according to a brightness of a localized region of image data, noise is suppressed while avoiding contrast reduction in the image data.

[Eleventh example]

Fig. 4, 28 or 32 is applied to a biological observation apparatus according to the eleventh example useful for understanding the present invention. In the configurations thereof, while a spatial frequency filter (LPF), not shown, is allocated inside the matrix computing section 436, control is performed so that spatial frequency characteristics thereof are slightly changed to, for example, widen a band.

The control section 42 changes a setting of characteristics (LPF characteristics) of a spatial frequency filter provided at the matrix computing section 436 in the main body processing apparatus (processor) 43. More specifically, the control section 42 performs control so that band characteristics of the LPF changes to that of a broadband during spectral image observation. Such a control operation is described in Fig. 4 of Japanese Patent Application No. 2004-250978.

Now, let us assume that the biological observation apparatus is currently in normal image observation mode.

In this state, an operator is able to perform endoscopy by inserting the insertion portion 102 of the endoscope 101 into a body cavity of a patient. When desiring to observe vascular travel or the like of the surface of an examination object tissue such as a diseased part or the like in the body cavity in greater detail, the operator operates a mode switching switch, not shown.

When the mode switching switch is operated, the control section 42 changes the operation modes of the light source section 41 and the main body processing apparatus 43 to a setting state of the spectral image observation mode.

More specifically, the control section 42 performs changing/setting such as: performing light quantity control so as to increase light quantity with respect to the light source section 41; changing the spatial frequency band characteristics of the LPF in the matrix computing section 436 to that of a broadband with respect to the main body processing apparatus 43; and controlling the switching section 439 to switch to the spectral image processing system that includes the matrix computing section 436 and the like.

By performing such changing/setting, travel of capillaries in the vicinity of surface layers of biological tissue can be displayed in a readily identifiable state during spectral image observation mode.

In addition, since band characteristics of signal passage through an LPF is changed to that of a broadband, resolution of travel of capillaries or vascular travel close to the vicinity of surface layers can be improved so as to equal the resolution of a color signal in a specific color G that is picked up under a G-colored illumination light, and an easily diagnosed image with good image quality can be obtained.

According to the present example that operates as described above, an existing synchronous color image pickup function can be retained in normal image observation mode, and, at the same time, even in spectral image observation mode, observation functions in spectral image observation mode can be sufficiently secured by changing processing characteristics such as changing the settings of coefficients or the like of the respective sections in the main body processing apparatus 43.

[Twelfth example]

As for a configuration of a biological observation apparatus according to the twelfth example useful for understanding the present invention, Fig. 4, 28 or 32 is applied. Additionally, in the configurations thereof, an NBI display indicating that spectral image observation is in progress is performed.

(1) Displaying on the display monitor 106

On the display monitor 106, nothing is displayed during normal image observation, while characters "NBI" are displayed during spectral image observation. Alternatively, instead of character display, a mark such as o may be displayed in, for example, one of the four corners of the monitor.

(2) Displaying on the front panel of the endoscope apparatus main body 105: refer to Figs. 39, 40 and 41

An LED is simply provided on the operating panel, and is turned off during normal image observation and turned on during spectral image observation. More specifically, as shown in Fig. 39, an LED lighting section 91 is provided in the vicinity of the characters "NBI" and is turned off during normal image observation and turned on during spectral image observation.

As shown in Fig. 40, an LED is provided so that either the characters "NBI" themselves 92 or a character periphery 93 instead of the characters "NBI" are lighted. Lighting is turned off during normal image observation and turned on during spectral image observation.

As shown in Fig. 41, an LED is provided so that either the characters "NBI" themselves 94 or a character periphery 95 instead of the characters "NBI" are lighted. Lighting is performed using different colors. For example, green is turned off during normal image observation and white is turned on during spectral image observation.

(3) Displaying on a centralized controller

A biological observation apparatus is assembled from a system including a plurality of devices, whereby display is performed on a screen of a controller that performs centralized control over the devices in the same manner as in Figs. 39, 40 and 41. Alternatively, a spectral image observation mode switching switch (i.e., NBI switch) itself is displayed in black characters during normal image observation and displayed in reversed characters during spectral image observation.

(4) Display locations other than the above include a keyboard and a foot switch.

[Thirteenth example]

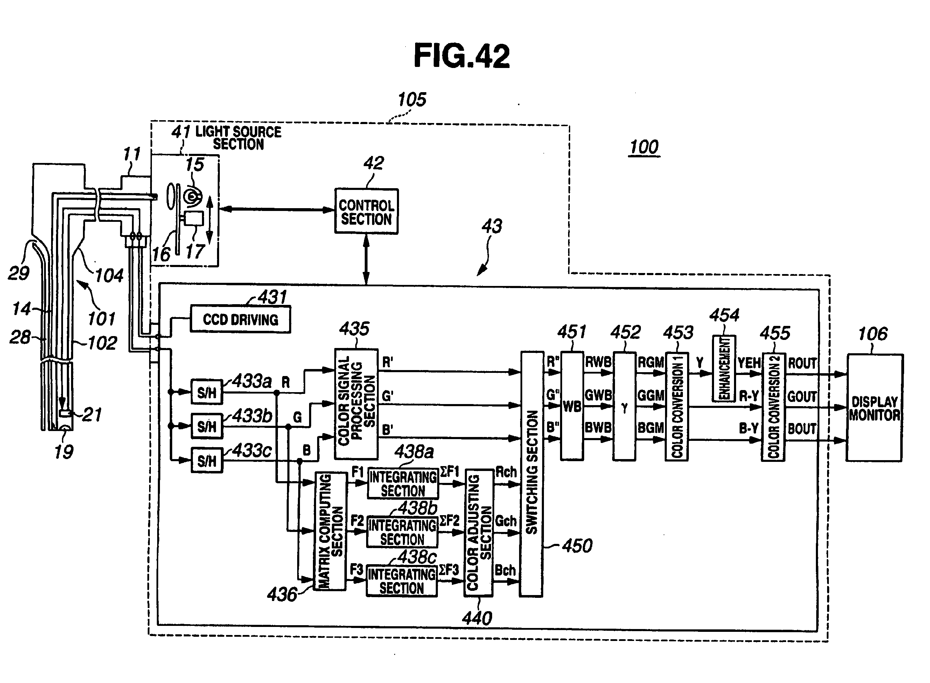

Fig. 42 is a block diagram showing a configuration of a biological observation apparatus according to a thirteenth example useful for understanding the present invention. Fig. 42 is a block diagram of a synchronous electronic endoscope apparatus 100.

As shown in Fig. 42, an endoscope apparatus main body 105 primarily comprises a light source unit 41, a control section 42, and a main body processing apparatus 43. Descriptions of like portions to those in the first example and shown in Fig. 4 are omitted, and the description below will focus on portions that differ from Fig. 4.

In Fig. 42, in the same manner as the light source section 41, the main body processing apparatus 43 is connected to the endoscope 101 via the connector 11. The main body processing apparatus 43 is provided with a CCD driving circuit 431 for driving the CCD 21. In addition, a color signal processing system is provided as a signal circuit system for obtaining normal images.

The color signal processing system comprises: sample-and-hold circuits (S/H circuits) 433a to 433c, connected to the CCD 21, which perform sampling and the like on a signal obtained by the CCD 21 and which create RGB signals; and a color signal processing section 435 connected to outputs of the S/H circuits 433a to 433c and which creates color signals R', G' and B'.

Color signals R', G' and B' are sent to common circuit sections (451 to 455) from the color signal processing section 435 via the switching section 450.

The signal processing of the circuits 451 to 455 is signal processing for displaying an image pickup signal that is a color image signal and a spectral signal created from the image pickup signal on the display monitor 106, and is capable of sharing between both image pickup signal processing and spectral signal processing.

Next, a description will be given on a configuration of the common circuit sections (451 to 455) which enable circuits for performing necessary signal processing including color adjustment processing such as white balance (hereinafter WB) processing, tone conversion processing such as $\gamma$ adjustment, spatial frequency enhancement processing such as contour correction to be shared while suppressing circuit size of the biological observation apparatus.

The common circuit sections (451 to 455) are configured so that WB processing, $\gamma$ processing and enhancement processing may be shared between normal observation images and spectral observation images.

In the present thirteenth example, as shown in Fig. 42, the following circuits a) to c) are arranged to be shared when creating normal observation images and spectral observation images. a) WB circuit 451, b) $\gamma$ correcting circuit 452, and c) enhancing circuit 454 are shared.

An output of the color adjusting section 440 and an output of the matrix computing section 436 are supplied via the switching section 450 to the WB circuit 451, the $\gamma$ correcting circuit 452 and the color converting circuit (1) 453 to create a Y signal, an R-Y signal and a B-Y signal. Then, an enhanced luminance signal YEH, an R-Y signal and a B-Y signal to be described later are further created and supplied to the color converting circuit (2) 455, and sent as R, G and B outputs to the display monitor 106.

Incidentally, as an example of quasi-bandpass filters F1 to F3, spectral images (F1, F2, and F3) from the matrix computing section 436 are created according to the following procedure.

F1: image with wavelength range between 520 nm to 560 nm (corresponding to G band)
F2: image with wavelength range between 400 nm to 440 nm (corresponding to B band)
F3: image with wavelength range between 400 nm to 440 nm (corresponding to B band)

Images resulting from integration processing and color adjustment processing performed on the above-mentioned spectral images (F1 to F3), as well as normal observation images (R', G' and B') are selected at the switching section 450 using a mode switching switch, not shown, provided on a front panel or a keyboard.

An output from the above-mentioned switching section 450 is subjected to processing by the WB circuit 451 and the $\gamma$ correcting circuit 452, and subsequently converted at the color converting circuit (1) 453 into a luminance signal (Y) and color difference signals (R-Y/B-Y).

Contour correction is performed by the enhancing circuit 454 on the afore-mentioned post-conversion luminance signal Y.

Subsequently, conversion to RGB is once again performed by the color converting circuit (2) 455, and output is performed to the display monitor 106.

The configuration of the present thirteenth example is advantageous in that: for normal observation images and spectral observation images, it is now possible to share and use WB/$\gamma$/enhancement processing; and since outputting spectral images (F1, F2, F3) from the matrix computing section 436 as G-B-B causes a luminance signal of a spectral image converted by the color converting circuit (1) 453 to include a high proportion of B components, it is now possible to focus on performing enhancement processing on superficial vascular images obtained from B spectral images.

Moreover, in the thirteenth example shown in Fig. 42, while a configuration in which primarily WB, $\gamma$ correction and enhancement processing is shared between the normal observation image system and the spectral observation image system, the present invention is not limited to this configuration. Alternatively, a configuration is possible in which at least one of WB, tone conversion and spatial frequency enhancement processing is shared.

According to the present example, a spectral image on which vascular patterns are clearly displayed can be obtained.

[Fourteenth example]

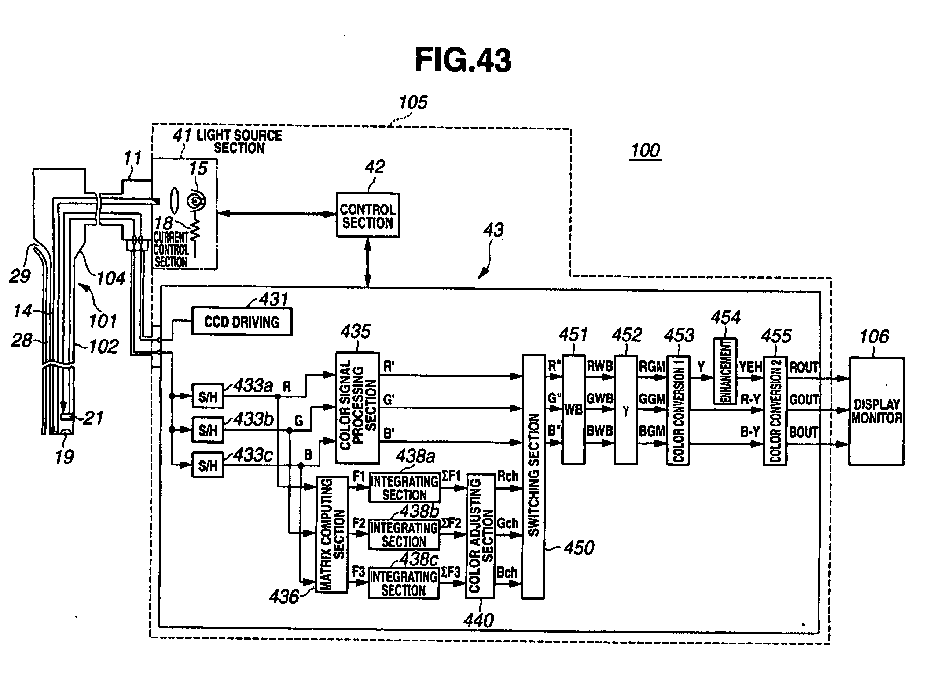

Fig. 43 is a block diagram showing a configuration of a biological observation apparatus according to a fourteenth example useful for understanding the present invention.

Since the fourteenth example is practically the same as the thirteenth example, only differences therefrom will be described. Like components will be assigned like reference characters and descriptions thereof will be omitted.

The present example primarily differs from the thirteenth example in the light source section 41 that performs illumination light quantity control. In the present example, control of light quantity irradiated from the light source section 41 is performed by controlling the current of the lamp 15 instead of by a chopper. More specifically, a current control section 18 as a light quantity control section is provided at the lamp 15 shown in Fig. 43.

As for operations of the present example, the control section 42 controls the current flowing through the lamp 15 so that neither of the color image signals of RGB reach a saturated state. Consequently, since the current used by the lamp 15 for emission is controlled, the light quantity thereof varies according to the magnitude of the current.

Incidentally, since other operations are the same as those in the first example, descriptions thereof will be omitted.

According to the present example, in the same manner as the thirteenth example, a spectral image on which vascular patterns are clearly displayed can be obtained. In addition, the present example is advantageous in that the control method thereof is simpler than the light quantity control method using a chopper as is the case in the thirteenth example.

[Fifteenth example]

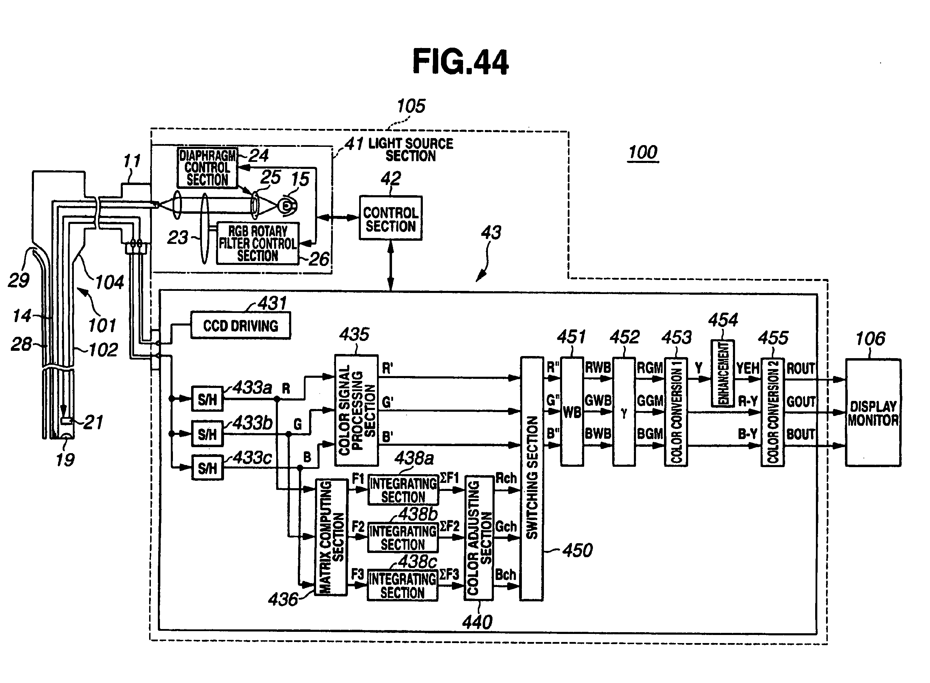

Fig. 44 is a block diagram showing a configuration of a biological observation apparatus according to a fifteenth example of the present invention. A diagram showing charge accumulation times of a CCD according to the example shown in Fig. 44 is the same as Fig. 33.

Since the fifteenth example is practically the same as the thirteenth example, only differences therefrom will be described. Like components will be assigned like reference characters and descriptions thereof will be omitted.

The present example primarily differs from the thirteenth example in the light source section 41 and the CCD 21. In the first example, the CCD 21 is provided with the color filters shown in Fig. 6 and is a so-called synchronous-type CCD that creates a color signal using the color filters. In contrast thereto, in the present fifteenth example, a so-called frame sequential-type is used which creates a color signal by illuminating illumination light in the order of R, G and B within a time period of a single frame.

As shown in Fig. 44, the light source section 41 according to the present example is provided with a diaphragm 25 that performs modulation on a front face of the lamp 15, and an RGB rotary filter 23 that makes, for example, one rotation during one frame is further provided on a front face of the diaphragm 25 in order to irradiate R, G and B frame sequential light. In addition, the diaphragm 25 is connected to a diaphragm control section 24 as a light quantity control section, and is arranged so as to be capable of performing modulation by limiting a light flux to be transmitted among light flux irradiated from the lamp 15 to change light quantity in response to a control signal from the diaphragm control section 24. Furthermore, the RGB rotary filter 23 is connected to an RGB rotary filter control section 26 and is rotated at a predetermined rotation speed.

As for operations by the light source section according to the present example, a light flux outputted from the lamp 15 is limited to a predetermined light quantity by the diaphragm 25. The light flux transmitted through the diaphragm 25 passes through the RGB rotary filter 23, and is outputted as respective illumination lights of R/GB at predetermined time intervals from the light source section. In addition, the respective illumination lights are reflected inside the subject to be examined and received by the CCD 21. Signals obtained at the CCD 21 are sorted according to irradiation time by a switching section (not shown) provided at the endoscope apparatus main body 105, and are respectively inputted to the S/H circuits 433a to 433c. In other words, when an illumination light is irradiated via the R filter from the light source section 41, a signal obtained by the CCD 21 is inputted to the S/H circuit 433a. Incidentally, since other operations are the same as those in the first embodiment, descriptions thereof will be omitted.

According to the present fifteenth embodiment, in the same manner as the thirteenth embodiment, a spectral image on which vascular patterns are clearly displayed can be obtained. In addition, unlike the thirteenth embodiment, the present fifteenth example is able to receive the full benefits of the so-called frame sequential method. Such benefits include, for example, those described in the modification shown in Fig. 34.

Furthermore, in the thirteenth example described above, illumination light quantity (light quantity from a light source) is controlled/adjusted in order to avoid saturation of R/G/B color signals. In contrast thereto, the present fifteenth example employs a method in which an electronic shutter of the CCD 21 is adjusted. At the CCD 21, charges accumulate in proportion to light intensity incident within a given time period, whereby the charge quantity is taken as a signal. What corresponds to the accumulation time is a so-called electronic shutter. By adjusting the electronic shutter by the CCD driving circuit 431, a charge accumulated quantity or, in other words, a signal quantity can be adjusted. As shown in Fig. 33, by obtaining RGB color images in a state where charge accumulation times are sequentially changed per one frame, a similar spectral image can be obtained. In other words, in each of the examples described above, illumination light quantity control by the diaphragm 25 may be used to obtain a normal image, and when obtaining a spectral image, it is possible to prevent saturation of R, G and B color images by varying the electronic shutter.

[Sixteenth example]

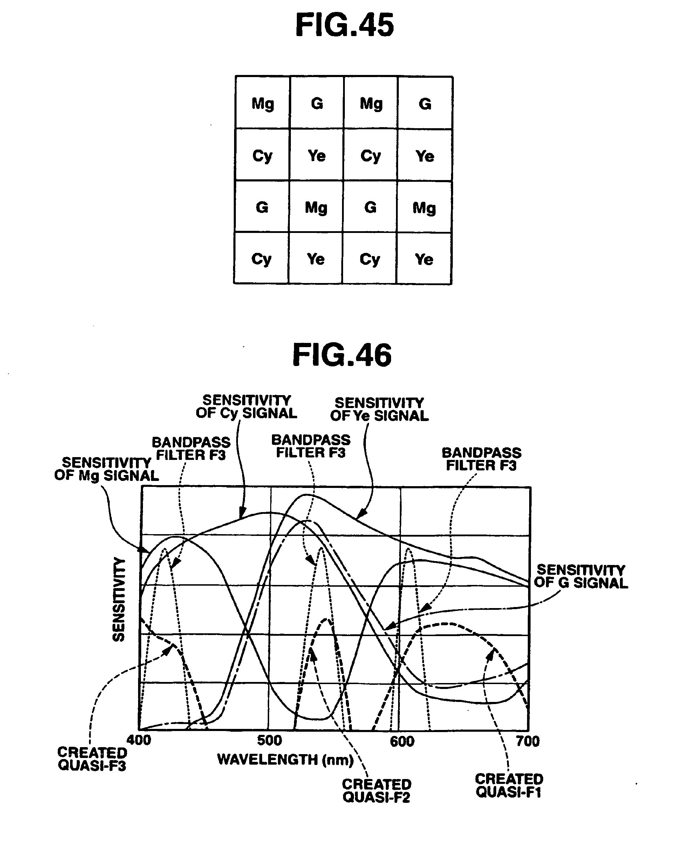

Figs. 45 and 46 relate to a biological observation apparatus according to a sixteenth example useful for understanding the present invention, wherein: Fig. 5 is a diagram showing an color filter array; and Fig. 46 is a diagram showing spectral sensitivity characteristics of the color filters shown in Fig. 45.

Since the biological observation apparatus at the thirteenth example is practically the same as the first example, only differences therefrom will be described. Like components will be assigned like reference characters and descriptions thereof will be omitted.

The present example primarily differs from the first example in the color filters provided at the CCD 21. Compared to the first example in which RGB primary color-type color filters are used as shown in Fig. 6, the present example uses complementary type color filters.

As shown in Fig. 45, the array of the complementary type filters is constituted by the respective elements of G, Mg, Ye and Cy. Incidentally, the respective elements of the primary color-type color filters and the respective elements of the complementary type color filters form relationships of Mg = R + B, Cy = G + B, and Ye = R + G.

In this case, it performs a full pixel readout from the CCD 21 and signal processing or image processing on the images from the respective color filters. In addition, by transforming Formulas 1 to 8 and 19 to 21 which accommodate primary color-type color filters so as to accommodate complementary type color filters, Formulas 27 to 33 presented below are derived. Note that target narrow bandpass filter characteristics are the same.

$$\begin{pmatrix} \mathbf{G} & \mathbf{Mg} & \mathbf{Cy} & \mathbf{Ye} \end{pmatrix} \begin{pmatrix} a_1 & a_2 & a_3 \\ b_1 & b_2 & b_3 \\ c_1 & c_2 & c_3 \\ d_1 & d_2 & d_3 \end{pmatrix} = \begin{pmatrix} \mathbf{F}_1 & \mathbf{F}_2 & \mathbf{F}_3 \end{pmatrix} \qquad \cdots (27)$$

$$\mathbf{C} = \begin{pmatrix} \mathbf{G} & \mathbf{Mg} & \mathbf{Cy} & \mathbf{Ye} \end{pmatrix} \qquad \mathbf{A} = \begin{pmatrix} a_1 & a_2 & a_3 \\ b_1 & b_2 & b_3 \\ c_1 & c_2 & c_3 \\ d_1 & d_2 & d_3 \end{pmatrix} \qquad \mathbf{F} = \begin{pmatrix} \mathbf{F}_1 & \mathbf{F}_2 & \mathbf{F}_3 \end{pmatrix} \qquad \cdots (28)$$

$$kG = \left( \int S(\lambda) \times H(\lambda) \times G(\lambda) d\lambda \right)^{-1}$$

$$kMg = \left( \int S(\lambda) \times H(\lambda) \times Mg(\lambda) d\lambda \right)^{-1}$$

$$kCy = \left( \int S(\lambda) \times H(\lambda) \times Cy(\lambda) d\lambda \right)^{-1}$$

$$kYe = \left( \int S(\lambda) \times H(\lambda) \times Ye(\lambda) d\lambda \right)^{-1} \qquad \cdots (29)$$

$$K = \begin{pmatrix} k_G & 0 & 0 & 0 \\ 0 & k_{Mg} & 0 & 0 \\ 0 & 0 & k_{Cy} & 0 \\ 0 & 0 & 0 & k_{Ye} \end{pmatrix} \quad \cdots(30)$$

$$A = \begin{pmatrix} -0.413 & -0.678 & 4.385 \\ -0.040 & -3.590 & 2.085 \\ -0.011 & -2.504 & -1.802 \\ 0.332 & 3.233 & -3.310 \end{pmatrix} \quad \cdots(31)$$

$$K = \begin{pmatrix} 1 & 0 & 0 & 0 \\ 0 & 0.814 & 0 & 0 \\ 0 & 0 & 0.730 & 0 \\ 0 & 0 & 0 & 0.598 \end{pmatrix} \quad \cdots(32)$$

$$A' = KA = \begin{pmatrix} 1 & 0 & 0 & 0 \\ 0 & 0.814 & 0 & 0 \\ 0 & 0 & 0.730 & 0 \\ 0 & 0 & 0 & 0.598 \end{pmatrix} \begin{pmatrix} -0.413 & -0.678 & 4.385 \\ -0.040 & -3.590 & 2.085 \\ -0.011 & -2.504 & -1.802 \\ 0.332 & 3.233 & -3.310 \end{pmatrix}$$

$$= \begin{pmatrix} -0.413 & -0.678 & 4.385 \\ -0.033 & -2.922 & 1.697 \\ -0.008 & -1.828 & -1.315 \\ 0.109 & 1.933 & -1.979 \end{pmatrix} \quad \cdots(33)$$

Furthermore, Fig. 46 shows spectral sensitivity characteristics when using complementary type color filters, target bandpass filters, and characteristics of quasi-bandpass filter determined from Formulas 27 to 33 provided above.

It is needless to say that, when using complementary type filters, the S/H circuits shown in Figs. 4, 42 are respectively applied to G/Mg/ Cy/Ye instead of R/GB.

According to the present example, in the same manner as in the first example, a spectral image capable of clearly displaying a vascular pattern can be obtained. In addition, the present example is able to receive the full benefit of using complementary type color filters.

While various examples according to the present invention have been described above, the present invention allows various combinations of the examples described above to be used. In addition, modifications may be made without departing from the scope thereof.

For example, for all previously described examples, the operator can create a new quasi-bandpass filter during clinical practice or at other timings and apply the filter to clinical use. In other words, with respect to the first example, a designing section (not shown) capable of computing/calculating matrix coefficients may be provided at the control section 42 shown in Figs. 4, 42.

Accordingly, a quasi-bandpass filter suitable for obtaining a spectral image desired by the operator may be arranged to be newly designed by inputting a condition via the keyboard provided on the endoscope apparatus main body 105 shown in Fig. 3. Accordingly, immediate clinical application can be achieved by setting a final matrix coefficient (corresponding to the respective elements of matrix <A'> in Formulas 21 and 33) derived by applying a correction coefficient (corresponding to the respective elements of matrix <K> in Formulas 20 and 32) to the calculated matrix coefficient (corresponding to the respective elements of matrix <A> in Formulas 19 and 31) to the matrix computing section 436 shown in Figs. 4, 42.

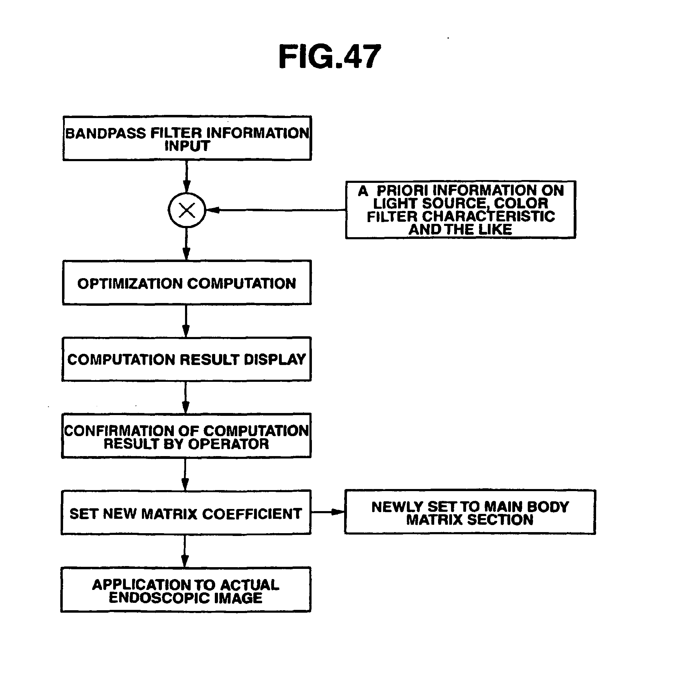

Fig. 47 shows a flow culminating in clinical application. To describe the flow in specific terms, first, the operator inputs information (e.g., wavelength band or the like) on a target bandpass filter via a keyboard or the like. In response thereto, a matrix <A'> is calculated together with characteristics of a light source, color filters of a CCD or the like stored in advance in a predetermined storage device or the like, and, as shown in Fig. 46, characteristics of the target bandpass filter as well as a computation result (quasi-bandpass filter) by the matrix <A'> are displayed on a monitor as spectrum diagrams.

After confirming the computation result, the operator performs settings accordingly when using the newly created matrix <A'>, and an actual endoscopic image is created using the matrix <A'>. At the same time, the newly created matrix <A'> is stored in a predetermined storage device, and can be reused in response to a predetermined operation by the operator.

As a result, irrespective of an existing matrix <A'>, the operator can create a new bandpass filter based on personal experience or the like. This is particularly effective when used for research purposes.

The present invention is not limited to the embodiments described above, and various changes and modifications may be made without departing from the scope thereof.

Industrial Applicability

The biological observation apparatus according to the present invention is particularly useful in applications in an electronic endoscope apparatus for acquiring biological information and performing detailed observations of biological tissue.

**Claims**

1. A biological observation apparatus comprising:

   an illuminating section (41) configured to irradiate light to a living body that is a subject to be examined;
   an image pickup section (21) configured to photoelectrically convert light reflected from the living body based on the irradiating light and to create an image pickup signal; and
   a signal processing control section (41, 42, 43) configured to control operations of the illuminating section and/or the image pickup section and to output the image pickup signal to a display device (106), wherein
   the signal processing control section includes:

      a light quantity control section (16) configured to control light quantity irradiated from the illuminating section;
      a spectral signal creating section (433a, 433b, 433c) configured to create a spectral signal corresponding to an optical wavelength narrowband image from the image pickup signal through signal processing;
      a color adjusting section (440) configured to, when outputting the spectral signal to the display device, allocate a different color tone for each of a plurality of bands forming the spectral signal;
      an image quality adjusting section (438a, 438b, 438c) configured to adjust image quality of a signal to be outputted to the display device; and
      a signal amplifying section configured to amplify a signal level of the image pickup signal and/or the spectral signal, wherein, between the image pickup signal and the spectral signal, the signal amplifying section is configured to vary amplification control performed thereon, and the amplification control is a follow-up speed upon commencement of an amplifying operation by the amplifying function when light quantity control by the light quantity control section becomes unavailable, and wherein the follow-up speed during spectral image observation is faster than the follow-up speed during the normal image observation.

2. The biological observation apparatus according to claim 1, wherein the signal amplifying section is controlled so as to operate in conjunction with light quantity control by the light quantity control section.

**Patentansprüche**

1.  Biologisches Beobachtungsgerät mit:

    einem Beleuchtungsbereich (41), der dazu ausgebildet ist, Licht auf einen lebendigen Körper zu strahlen, der ein zu untersuchender Patient ist;
    einem Bildaufnahmebereich (21), der dazu ausgebildet ist, Licht, das auf der Basis des ausgestrahlten Lichts von dem lebendigen Körper reflektiert wird, photoelektrisch umzuwandeln, und ein Bildaufnahmesignal zu erzeugen; und
    einem Signalverarbeitungssteuerbereich (41, 42, 43), der dazu ausgebildet ist, Operationen des Beleuchtungsbereichs und/oder des Bildaufnahmebereichs zu steuern und das Bildaufnahmesignal an eine Anzeigevorrichtung (106) auszugeben, wobei
    der Signalverarbeitungssteuerbereich umfasst:

    einen Lichtmengensteuerbereich (16), der dazu ausgebildet ist, die Lichtmenge zu steuern, die von dem Beleuchtungsbereich ausgestrahlt wird;
    einen Spektralsignalerzeugungsabschnitt (433a, 433b, 433c), der dazu ausgebildet ist, durch Signalverarbeitung aus dem Bildaufnahmesignal ein Spektralsignal zu erzeugen, das einem hinsichtlich der optischen Wellenlänge schmalbandigen Bild entspricht;
    einen Farbeinstellungsbereich (440), der dazu ausgebildet ist, wenn das Spektralsignal an die Anzeigevorrichtung ausgegeben wird, jedem einer Mehrzahl von Bändern, die das Spektralsignal bilden, einen unterschiedlichen Farbton zuzuordnen;
    einen Bildqualitätseinstellungsbereich (438a, 438b, 438c), der dazu ausgebildet ist, die Bildqualität eines Signals einzustellen, das an die Anzeigevorrichtung ausgegeben werden soll; und
    einen Signalverstärkungsbereich, der dazu ausgebildet ist, einen Signalpegel des Bildaufnahmesignals und/oder des Spektralsignals zu verstärken, wobei der Signalverstärkungsbereich dazu ausgebildet ist, die auf das Bildaufnahmesignal und das Spektralsignal ausgeübte Verstärkungssteuerung zwischen den beiden zu variieren, und auf den Beginn einer Verstärkungsoperation durch die Verstärkungsfunktion hin, wenn die Lichtmengensteuerung durch den Lichtmengensteuerbereich nicht mehr verfügbar ist, die Verstärkungssteuerung eine Nachfolgegeschwindigkeit ist, und wobei die Nachfolgegeschwindigkeit während einer Spektralbildbeobachtung schneller ist als die Nachfolgegeschwindigkeit während der normalen Bildbeobachtung.

2.  Biologische Beobachtungsvorrichtung nach Anspruch 1, wobei der Signalverstärkungsbereich so gesteuert wird, dass er in Verbindung mit der Lichtmengensteuerung durch den Lichtmengensteuerbereich arbeitet.

**Revendications**

1.  Appareil d'observation biologique comprenant:

    une section d'éclairage (41) configurée pour projeter une lumière sur un corps vivant qui est un sujet devant être examiné;
    un section de capture d'image (21) configurée pour convertir photo-électriquement une lumière réfléchie du corps vivant sur la base de la lumière de projection et pour créer un signal de capture d'image; et
    une section de commande de traitement de signal (41, 42, 43) configurée pour commander des opérations de la section d'éclairage et/ou de la section de capture d'image et pour délivrer en sortie le signal de capture d'image jusqu'à un dispositif d'affichage (106), dans lequel
    la section de commande de traitement de signal inclut:

    une section de commande de quantité de lumière (16) configurée pour commander une quantité de lumière projetée de la section d'éclairage;
    une section de création de signal spectral (433a, 433b, 433c) configurée pour créer un signal spectral correspondant à une image en bande étroite de longueur d'onde optique à partir du signal de capture d'image par l'intermédiaire d'un traitement de signal;

une section d'ajustement de couleur (440) configurée pour, lors de la délivrance en sortie du signal spectral jusqu'au dispositif d'affichage, attribuer une tonalité de couleur différente pour chacune d'une pluralité de bandes formant le signal spectral;

une section d'ajustement de qualité d'image (438a, 438b, 438c) configurée pour ajuster une qualité d'image d'un signal devant être délivré en sortie jusqu'au dispositif d'affichage; et

une section d'amplification de signal configurée pour amplifier un niveau de signal du signal de capture d'image et/ou du signal spectral, dans lequel, entre le signal de capture d'image et le signal spectral, la section d'amplification de signal est configurée pour faire varier une commande d'amplification exécutée sur celui-ci, et la commande d'amplification est une vitesse de suivi au commencement d'une opération d'amplification par la fonction d'amplification lorsque la commande de quantité de lumière par la section de commande de quantité de lumière devient indisponible, et dans lequel la vitesse de suivi pendant une observation d'image spectrale est plus rapide que la vitesse de suivi pendant l'observation d'image normale.

2. Appareil d'observation biologique selon la revendication 1, dans lequel la section d'amplification de signal est commandée de manière à fonctionner en conjonction avec une commande de quantité de lumière par la section de commande de quantité de lumière.

# FIG.1

# FIG.2

*438a(438b, 438c)*

FROM MATRIX
COMPUTING SECTION ──────────────→ | INTEGRATING SECTION | ──────────────→ TO COLOR ADJUSTING
436                                                                            SECTION 440

IRRADIATED WHITE
LIGHT SENSITIVITY:
I0

IRRADIATED WHITE
LIGHT SENSITIVITY:
I1

IRRADIATED WHITE
LIGHT SENSITIVITY:
In

INTEGRATED SPECTRAL
SENSITIVITY

EP 2 332 460 B1

# FIG.3

# FIG.4

EP 2 332 460 B1

# FIG.5

# FIG.6

| R | G | R | G |
|---|---|---|---|
| R | B | R | B |
| R | G | R | G |
| R | B | R | B |

# FIG.7

# FIG.8

# FIG.9

S(λ)

SENSITIVITY

400      500      600      700

WAVELENGTH (nm)

# FIG.10

H(λ)

SENSITIVITY

400      500      600      700

WAVELENGTH (nm)

# FIG.11

SUPERFICIAL
IRREGULAR STRUCTURE

CAPILLARY NETWORK
NEAR SURFACE

VASCULAR NETWORK
NEAR INTERMEDIATE
LAYERS

NETWORK OF LARGE
VEINS NEAR DEEP
LAYERS

46     45

47

48

**FIG.12**

**FIG.13**

**FIG.14**

**FIG.15**

**FIG.16**

**FIG.17**

LIGHT QUANTITY

F3 F2 F1 WAVELENGTH

380 700

**FIG.18**

**FIG.19**

EP 2 332 460 B1

**FIG.20**

**FIG.21**

62a  61  63a

LUT → 3 BY 3 MATRIX CIRCUIT → LUT  440

62b  63b

LUT → LUT  440a

62c  63c

LUT → LUT

64

COEFFICIENT CHANGING CIRCUIT

FROM PROCESSING CONVERSION SWITCH

**FIG.22**

BLUE PATTERN

RED PATTERN

YELLOW PATTERN

# FIG.23

FROM PROCESSING
CONVERSION SWITCH

# FIG.24

# FIG.25

# FIG.26

# FIG.27

FIG.28

# FIG.29

*66*

*67*

*68*

*69  71  70*

# FIG.30

*66A*

*67*

*68*

*69*

# FIG.31

# FIG.32

EP 2 332 460 B1

# FIG.33

# FIG.34

**FIG.35**

# FIG.36

# FIG.37

# FIG.38

# FIG.39

○─ 91
NBI

# FIG.40

─ 93
NBI ─ 92

# FIG.41

─ 95
NBI ─ 94

**FIG.42**

FIG.43

# FIG.44

EP 2 332 460 B1

# FIG.45

| Mg | G | Mg | G |
|----|----|----|----|
| Cy | Ye | Cy | Ye |
| G | Mg | G | Mg |
| Cy | Ye | Cy | Ye |

# FIG.46

SENSITIVITY OF Cy SIGNAL

SENSITIVITY OF Ye SIGNAL

BANDPASS FILTER F3

BANDPASS FILTER F3

BANDPASS FILTER F3

SENSITIVITY OF Mg SIGNAL

SENSITIVITY OF G SIGNAL

SENSITIVITY

400    500    600    700

WAVELENGTH (nm)

CREATED QUASI-F3

CREATED QUASI-F2

CREATED QUASI-F1

# FIG.47

```
┌─────────────────────────────┐
│ BANDPASS FILTER INFORMATION │
│           INPUT             │
└─────────────────────────────┘
              │
              ▼
           ╭─────╮           ┌──────────────────────────────┐
           │  ✕  │◄──────────│  A  PRIORI INFORMATION ON    │
           ╰─────╯           │   LIGHT SOURCE, COLOR        │
              │              │  FILTER CHARACTERISTIC       │
              │              │      AND THE LIKE            │
              ▼              └──────────────────────────────┘
┌─────────────────────────────┐
│   OPTIMIZATION COMPUTATION   │
└─────────────────────────────┘
              │
              ▼
┌─────────────────────────────┐
│ COMPUTATION RESULT DISPLAY   │
└─────────────────────────────┘
              │
              ▼
┌─────────────────────────────┐
│ CONFIRMATION OF COMPUTATION  │
│      RESULT BY OPERATOR      │
└─────────────────────────────┘
              │
              ▼
┌─────────────────────────────┐     ┌──────────────────────────────┐
│ SET NEW MATRIX COEFFICIENT   │────►│   NEWLY SET TO MAIN BODY     │
└─────────────────────────────┘     │      MATRIX SECTION          │
              │                      └──────────────────────────────┘
              ▼
┌─────────────────────────────┐
│    APPLICATION TO ACTUAL     │
│     ENDOSCOPIC IMAGE         │
└─────────────────────────────┘
```

**EP 2 332 460 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2002095635 A
- JP 2003093336 A
- US 20030158470 A1
- US 5408263 A
- US 5868666 A
- JP 2005082544 A
- JP 2004250978 A